# EUROPEAN PATENT SPECIFICATION

(11) **EP 4 606 410 B1**
(45) Date of publication and mention of the grant of the patent: **25.11.2026**
(21) Application number: 23890421.3
(22) Date of filing: 28.09.2023
(51) Int. Cl.: A61M 16/04

(54) **AIRWAY MANAGEMENT SYSTEM**
ATEMWEGSVERWALTUNGSSYSTEM
SYSTÈME DE GESTION DES VOIES RESPIRATOIRES

(30) Priority: 15.11.2022 CN 202211428717
(43) Date of publication of application: 27.08.2025
(73) Proprietor: Yang, Ming, Tianjin 300051 (CN)
(72) Inventor: Yang, Ming, Tianjin 300051 (CN)
(74) Representative: Murgitroyd & Company
(86) International application number: PCT/CN2023/122380
(87) International publication number: WO 2024/103984

(56) References cited:
- WO-A1-2020/106666
- WO-A1-2020/150775
- WO-A2-02/40079
- WO-A2-02/40079
- WO-A2-2014/058840
- CN-A- 103 463 719
- CN-A- 103 599 583
- CN-A- 104 800 941
- CN-A- 107 823 767
- CN-A- 107 899 118
- CN-A- 115 738 005
- CN-U- 206 837 214
- US-A1- 2005 139 220
- US-A1- 2014 276 178
- US-A1- 2016 256 648
- US-A1- 2016 262 603
- US-A1- 2020 001 032

## Description

### TECHNICAL FIELD

This invention relates to the field of clinical medical device technology, specifically relating to an airway management system.

### BACKGROUND

An airway management system will be typically used on patients during the administration of general anesthesia or during treatment in an intensive care unit (ICU). The airway management system typically comprises an anaesthetic mask, a laryngeal mask, an endotracheal tube, a connecting tube, a telescopic tube, a heat and moisture exchanger (HME), a breathing circuit and the like. The airway management system can achieve functions such as general anesthesia, respiratory support, detection of inhaled or exhaled gas composition, delivery of oxygen and anesthetic gases, and discharge of respiratory waste gases. The respiratory patterns for the patients include: both spontaneous respiration and apparatus-controlled ventilation.

Due to the influence of own constitutions of the patients, drugs used and underlying diseases, there are significant differences in tidal volume, respiratory rate, and minute ventilation of patients who retain spontaneous respiration. Smaller tidal volume, respiratory rate, minute ventilation, and airway pressure should be selected as possible for patients with apparatus-controlled ventilation on the premise of satisfying gas exchange. Every time the patient inhales, he/she will inhale part of the exhaled gas. Moreover, the same patient often experiences the two respiratory patterns. An existing airway management system has a common space for the inhaled gas and the exhaled gas, which cannot meet various different respiratory patterns of the patients. When the tidal volume of the spontaneous respiration of the patient is high, the airway resistance will increase. When the tidal volume of the spontaneous respiration of the patient is small or protective apparatus-controlled ventilation is administrated, ineffective ventilation will increase, and repeated inhalation is more frequent, which influences gas exchange. A patient with severe pneumonia requires invasive ventilation due to fact that an increase in anatomic dead space results in a decrease in blood oxygen content and an increase in blood carbon dioxide content, that is, the patient is connected to a ventilator via the breathing circuit after tracheal intubation. There are several reasons for the increase in anatomic dead space of the patient: 1. inflammatory exudates fill alveoli; 2. the blood flow ratio for supine position ventilation is imbalanced; and 3. apparatus ventilation causes the increase in anatomic dead space. An existing solution is prone position ventilation for the patients, and the prone position ventilation can reduce the anatomic dead space of the patient, and corrects the imbalance of the blood flow ratio of ventilation. Prone position ventilation treatment is not commonly used in clinical practice due to the following drawbacks: 1. medical and nursing resources are overstretched; 2. the risk of airway obstruction is increased; and 3. the risk of pressure sores and head and face injuries in patients are increased.

Due to the limitation of the internal diameter of a ventilation tube, only an endotracheal tube with an internal diameter of less than 7.0 mm can be inserted into a conventional intubating laryngeal mask. An endotracheal tube with an average internal diameter of 7.5 mm should be selected for female patients in China, and an endotracheal tube with an average internal diameter of 8.5 mm should be selected for male patients in China. If the endotracheal tube with the internal diameter of less than 7.0 mm is selected, there may be the following hazards in clinical practice: 1. the artificial airway resistance is increased, which increases respiratory and circulatory system complications; 2. The cuff pressure of the endotracheal tube is increased, which causes injure to the mucosa of a tracheal lining. 3. Operations by a ventilation cavity of the endotracheal tube will be limited, comprising insertion of a bronchial occluder, examination and treatment under a fiberoptic bronchoscope, etc.

The tail end of a conventional gas-sampling line is provided in an apparatus dead space of an artificial airway, and its position is not fixed. Since a sampling site is far away from alveoli and interfered by the exhaled exhaust gas of the patient, a numerical value obtained by sampling analysis is delayed and inaccurate.

A conventional inner tube has a smooth outer wall and is cylindrical, and is prone to adherence after being inserted into the laryngeal mask and a lumen of the endotracheal tube, thereby causing obstruction between inner and outer tubes.

A conventional connecting tube is of an equal diameter structure and cannot be connected to a patient end of a breathing circuit connector in this invention.

A conventional breathing circuit connector is L-shaped, Y-shaped or used in combination, and cannot be applied in this invention.

WO02/40079A2 discloses a laryngeal mask airway comprising a curved tubular guide for insertion through a patient's mouth and into the patient's airway, said guide having a distal opening to abut the patient's laryngeal inlet and a proximal opening remaining outside the patient's mouth after insertion of the guide, said guide allowing insertion of an endotracheal tube along the guide to a position past the patient's larynx; a laryngeal mask surrounding the distal opening of the guide to substantially seal the laryngeal inlet about the distal opening of the guide; and a ventilation port adjacent to the proximal opening of the guide to supply a flow of air/oxygen through the guide and the patient's larynx into the patient's lungs during insertion of an endotracheal tube along the guide.

US2020/001032A1 discloses a three-way connecting tube comprising a proximal end configured and dimensioned to couple to an anesthesia circuit; a first branch configured and dimensioned to be connected to an airway channel of a laryngeal mask, the first branch including a pressure relief valve to limit a pressure to the laryngeal mask while ensuring a higher pressure to a bronchial isolation tube; a second branch configured and dimensioned to be connected to the bronchial isolation tube; and a built-in thermo-moisture filter and a CO2 sampling port.

US2016/256648A1 discloses an airway device capable of insertion into the throat of a patient to open the airway of the patient, the airway device comprising at least one tube formed of a compliant plastic material insertable into the throat of the patient; a camera lumen attached to the at least one tube, the camera lumen having a distal end and an open proximal end; a clear window attached to and sealing the distal end of the camera; and a camera insertable through the open proximal end such that the camera is proximate to the clear window and removable from the camera lumen through the open proximal end.

US2016/262603A1 discloses an oral airway device comprising a two-part tubal body of adjustable length, with a lumen and open proximal and distal ends, wherein the two-part tubal body is made from an inner cylindrical tube at least partially inserted into an outer cylindrical tube, wherein the device further comprises a bougie and a visualization device comprising a camera tube, and a camera placed in the camera tube, wherein the distal end of the camera tube is sealed with a transparent material which creates a sealed distal transparent window.

US2005/139220A1 discloses a method for guiding insertion of an endotracheal tube into the trachea of a patient while continuing resuscitation of the patient, comprising the steps of: - inserting a curved guide through a patient's mouth and hypopharynx; - resuscitating the patient by supplying air/oxygen; - applying a series of cardiac chest compressions to the patient while resuscitation continues; - advancing the endotracheal tube along the guide into the patient's trachea while resuscitation with delivery of air/oxygen and cardiac chest compressions continue; and - ventilating the patient through the endotracheal tube.

### SUMMARY

To solve technical problems in the prior art, an airway management system is provided. The invention is defined in the appended independent claims 1 and 2. Preferred embodiments are matter of the dependent claims.

In clinical use, the airway management system can reduce apparatus dead space and improve the sensitivity and accuracy of gas sampling analysis while enabling stable ventilation connections.

In one implementation, the airway management system comprises a laryngeal mask component having a first ventilation cavity and an inner tube component receivable in the first ventilation cavity.

In one implementation, the airway management system further comprises a breathing circuit connector having, at a patient end, a patient end outer connector configured to connect to a laryngeal mask connector and a patient end inner connector configured to connect to an inner tube connector, and having an opposite end configured to connect to an anesthesia machine or a ventilator through a breathing circuit.

One embodiment includes a telescopic tube component for guiding insertion, gas sampling and throat aspiration cavities arranged in the laryngeal mask airway tube, an unequal-diameter connecting tube for anesthesia induction, a multi-channel machine end of the breathing circuit connector with an openable top end cover and a Luer taper, protrusions on the outer wall of the inner tube body for positioning, and additional cavities such as a drainage cavity and/or a video component cavity.

Patients typically use an airway management system during general anesthesia implementation or during treatment in the Intensive Care Unit (ICU). The airway management system typically comprises an anaesthetic mask, a laryngeal mask, an endotracheal tube, a connecting tube, a telescopic tube, a heat and moisture exchanger (HME), a breathing circuit and the like. The airway management system can achieve functions such as general anesthesia, respiratory support, detection of inhaled or exhaled gas components, delivery of oxygen and anesthetic gases, and discharge of respiratory waste gases. The respiratory patterns for the patients include two types: spontaneous respiration and apparatus-controlled ventilation. A patient end of a gas sampling cavity of the laryngeal mask component or the endotracheal tube component and the gas sampling cavity are located on the ventral side of the patient, and the patient end of the gas sampling cavity is close to a patient end of a tube, which makes sampling analysis more sensitive and accurate. A patient end of a throat aspiration cavity of the laryngeal mask component or the endotracheal tube component and the throat aspiration cavity are located on the dorsal side of the patient; the patient end of the throat aspiration cavity is close to the patient end of the catheter; and the throat aspiration cavity can aspirate secretions near the glottis of the patient and or may serve as a standby gas sampling cavity. The gas sampling cavity and the throat aspiration cavity are fixedly mated, and are symmetrically provided, which guarantees the continuity and reliability of gas sampling analysis. The laryngeal mask airway connector can be connected to a patient end of a telescopic tube component; when the telescopic tube component is fully extended to its completely open state, the inner tube component can be inserted via the laryngeal mask component and the telescopic tube component; a patient end of the inner tube component is located on the glottis, and in a mask body of the laryngeal mask component, and is aligned with a patient end of a laryngeal mask airway tube, which can eliminate apparatus dead space, avoid that a patient inhales his/her own exhaled exhaust gas, and can improve the sensitivity and accuracy of results of gas sampling analysis in the airway of the patient. As the telescopic tube component gradually contracts, the patient end of the inner tube component moves towards the lungs of the patient, passes through the glottis, and enters the trachea; and when the telescopic tube component contracts until it is in its original state, the patient end of the inner tube component reaches the trachea, thereby eliminating all the apparatus dead space and some of anatomic dead space. The inner tube connector and the telescopic tube machine end connector are jointly connected to the patient end of the breathing circuit connector, and the breathing circuit connector is connected to an anesthesia machine or a ventilator through a breathing circuit.

The purpose of the first aspect of this invention is to provide an airway management system to solve the technical problem of difficulty in eliminating apparatus dead space in existing system.

The airway management system provided in the first aspect of this invention comprising a laryngeal mask component and an inner tube component, the inner tube component comprises an inner tube body, and a third ventilation cavity is arranged inside the inner tube body; where the laryngeal mask component comprises a mask body, a laryngeal mask airway tube connected to one end of the mask body, a first ventilation cavity is arranged inside the laryngeal mask airway tube, and the first ventilation cavity is configured for inserting the inner tube component, after inserting the inner tube component, between the first ventilation cavity and the third ventilation cavity, one forms an inhalation cavity and another forms an exhalation cavity;

The patient end of the inner tube component is configured to be positioned within the mask body, and aligned with the patient end of the laryngeal mask airway tube; The patient end of the inner tube component can also be configured to enter the trachea of the patient through the glottis of the patient.

The beneficial effects of the airway management system provided in this invention are:

By inserting the inner tube component into the laryngeal mask component, the inner tube connector and the laryngeal mask airway connector jointly connect to the patient end of the breathing circuit connector, where the breathing circuit connector is connected to an anesthesia machine or a ventilator through a breathing circuit, which can eliminate apparatus dead space and avoid that the patient inhales his/her own exhaled exhaust gas.

The apparatus dead space and some of the anatomic dead space are eliminated, which has clinical significance for patients who require low tidal volume and low-driving pressure ventilation. The patients who require low tidal volume and low-driving pressure ventilation comprise but are not limited to: 1 patients undergoing pediatric surgery; 2 patients undergoing laparoscopic surgery that require carbon dioxide filling; 3 patients undergoing thoracic surgery; 4 patients suffering from pulmonary diseases and undergoing general anesthesia; 5 patients undergoing massive transfusion and infusion surgery; 6 patients undergoing prolonged surgery; 7 patients with chronic obstructive pulmonary disease (COPD) or acute respiratory distress syndrome (ARDS); and 8 patients with severe pneumonia who need invasive ventilation treatment.

For ARDS patients, this application can achieve a good therapeutic effect in the supine position, and will achieve a better therapeutic effect if combined with the prone position ventilation treatment.

Different specifications of laryngeal mask components or endotracheal tube components of this application can be used in combination with different specifications of inner tube components to meet the needs of patients with different glottic areas and different ventilator parameter settings.

In one embodiment, between the ventilation cavity formed after the first ventilation cavity inserted into the inner tube component and the third ventilation cavity, one forms an inhalation cavity and another forms an exhalation cavity; or
between the part of the first ventilation cavity inserted into the inner tube component and the third ventilation cavity, one forms an inhalation cavity and another forms an exhalation cavity.

In one embodiment, the laryngeal mask component further comprises a laryngeal mask airway connector, where the other end of the laryngeal mask airway tube is connected to the laryngeal mask airway connector; The inner tube component also comprises an inner tube connector connected to the inner tube body; The airway management system also comprises a breathing circuit connector component; the breathing circuit connector component comprises a breathing circuit connector, a breathing circuit connector top end cover, and a first Luer taper; one end of the breathing circuit connector is connected to the laryngeal mask airway connector, or connected to the laryngeal mask airway connector and the inner tube connector, and the other end of the breathing circuit connector is configured to connect to an anesthesia machine or a ventilator through a breathing circuit;

The breathing circuit connector comprises a patient end of the breathing circuit connector and a machine end of the breathing circuit connector; the patient end of the breathing circuit connector comprises a patient end inner connector and a patient end outer connector, wherein the patient end outer connector is configured to connect to the laryngeal mask airway connector, the patient end inner connector is configured to connect to the inner tube connector.

The laryngeal mask airway connector is connected to the patient end of the breathing circuit connector, and at this time, the breathing circuit connector top end cover is placed on the machine end first inner connector of the breathing circuit connector; By inserting a machine end second inner connector into the inner tube body, the inner tube connector of the inner tube component and the machine end outer connector of the breathing circuit connector can be connected to the patient end of the breathing circuit respectively, where the machine end of the breathing circuit is connected to an anesthesia machine or a ventilator; as the inner tube body is gradually inserted, the apparatus dead space in the airway management system gradually decrease; when the patient end of the inner tube component is located in the mask body of the laryngeal mask component, and is aligned with the patient end of the laryngeal mask airway tube, the apparatus dead space can be eliminated; at this time, the patient end of the inner tube component passes through the glottis of the patient and enters the trachea of the patient, thereby eliminating all the apparatus dead space and some of the anatomic dead space.

The laryngeal mask airway tube and the laryngeal mask airway connector have internal diameters larger than that of the existing product, which can reduce the airway resistance, implant a diagnosis and treatment device with a larger external diameter and insert an endotracheal tube with a larger external diameter via the laryngeal mask airway tube and the laryngeal mask airway connector. A sampling site of the laryngeal mask component is close to the patient end of the catheter, which can make the sampling site close to the alveoli to the greatest extent, such that the sampling analysis is more sensitive and accurate, and a sampling analysis numerical value and an arterial blood gas analysis numerical value are more approximate and have good correlation.

In one embodiment, the airway management system further comprises a telescopic tube component, the telescopic tube component comprises a telescopic tube patient end connector, a telescopic tube machine end connector, and a telescopic tube body, one end of the telescopic tube body is connected to the telescopic tube patient end connector, the other end of the telescopic tube body is connected to the telescopic tube machine end connector; the telescopic tube patient end connector is configured to connect the laryngeal mask airway connector; the inner tube component is configured to be inserted via the laryngeal mask airway tube, the laryngeal mask airway connector and the telescopic tube component; the telescopic tube machine end connector and the inner tube connector are configured to connect to the patient end of the breathing circuit connector;

When the telescopic tube component is configured to fully extend to its completely open state, the patient end of the inner tube component reaches the trachea of the patient; when the telescopic tube component gradually contracts, the patient end of the inner tube component moves towards the lungs of the patient, passes through the glottis of the patient, and enters the trachea of the patient.

The laryngeal mask airway connector can be connected to the patient end of the telescopic tube component; when the telescopic tube component is fully extended to its completely open state; the inner tube component can be inserted via the laryngeal mask component and the telescopic tube component; the patient end of the inner tube component is located on the glottis of the patient, and in the mask body of the laryngeal mask component, and is aligned with the patient end of the laryngeal mask airway tube, the inner tube connector and the telescopic tube machine end connector are jointly connected to the patient end of the breathing circuit connector, and the breathing circuit connector is connected to the anesthesia machine or the ventilator through the breathing circuit, which can eliminate apparatus dead space, avoid that a patient inhales his/her own exhaled exhaust gas from the laryngeal mask component, and can improve the sensitivity and accuracy of gas sampling analysis in the airway of the patient. As the telescopic tube component gradually contracts, the patient end of the inner tube component moves towards the lungs of the patient, passes through the glottis of the patient, and enters the trachea; when the telescopic tube component contracts until it is in its original state, the patient end of the inner tube component can be made reach the trachea of the patient, thereby eliminating all the apparatus dead space and some of anatomic dead space; and the inner tube connector and the telescopic tube machine end connector are jointly connected to the patient end of the breathing circuit connector, and the breathing circuit connector is connected to the anesthesia machine or the ventilator through the breathing circuit.

After the inner tube component is inserted via the laryngeal mask component and telescopic tube component, the patient end of the inner tube component can reach the trachea of the patient. Compared to an existing laryngeal mask product, a third ventilation cavity of the inner tube component can be selected as an inhalation channel, and a gap between the inner tube body and the trachea, the glottis, and the laryngeal mask airway tube may serve as an exhalation channel, which guarantees smooth respiration, reduces airway pressure, and lowers the risk of accidental aspiration. Due to the ventilation and positioning function of the inner tube component, the risk of airway obstruction is eliminated, and the stability of an artificial airway is increased. Compared to an existing endotracheal tube product, the risk of the mucosa of a tracheal lining is compressed after the cuff is inflated to cause mucosal ischemic necrosis is eliminated.

In an embodiment, the telescopic tube component further comprises: annular protrusions and a stopper; the annular protrusions are provided on outer side walls of the telescopic tube patient end connector and the telescopic tube machine end connector, respectively; the stopper is configured to limit the telescopic tube component; and when the telescopic tube component is fully extended to its completely open state, the stopper is located between the annular protrusions.

The laryngeal mask airway connector can be connected to the patient end of the telescopic tube component; when the telescopic tube component is fully extended to its completely open state, an optional way is to place the stopper between the annular protrusions; the inner tube component can be inserted via the laryngeal mask component and the telescopic tube component; the patient end of the inner tube component is located on the glottis of the patient, and in the mask body of the laryngeal mask component, and is aligned with the patient end of the laryngeal mask airway tube, the inner tube connector and the telescopic tube machine end connector are jointly connected to the patient end of the breathing circuit connector, and the breathing circuit connector is connected to the anesthesia machine or the ventilator through the breathing circuit, which can eliminate apparatus dead space, avoid that a patient inhales his/her own exhaled exhaust gas, and can improve the sensitivity and accuracy of gas sampling analysis in the airway of the patient. The annular protrusions of the telescopic tube component of this invention are provided on the outer side walls of the telescopic tube patient end connector and the telescopic tube machine end connector, respectively; the stopper is configured to limit the telescopic tube component; and when the telescopic tube component is fully extended to its completely open state, the stopper is located between the annular protrusions, so that the telescopic tube component cannot contract. This avoids that the patient end of the inner tube component injures the glottis and trachea of the patient due to the fact that the telescopic tube component contracts. After the stopper is removed, as the telescopic tube component gradually contracts, the patient end of the inner tube component moves towards the lungs of the patient, passes through the glottis of the patient, and enters the trachea; when the telescopic tube component contracts until it is in its original state, the patient end of the inner tube component can be made reach the trachea of the patient, thereby eliminating all the apparatus dead space and some of anatomic dead space; and the inner tube connector and the telescopic tube machine end connector are jointly connected to the patient end of the breathing circuit connector, and the breathing circuit connector is connected to the anesthesia machine or the ventilator through the breathing circuit.

The laryngeal mask component is connected to the patient end of the breathing circuit connector, and at this time, the breathing circuit connector top end cover is placed on the machine end first inner connector of the breathing circuit connector; the inner tube body of the inner tube component is inserted through the machine end second inner connector of the breathing circuit connector, so that the inner tube connector and the machine end outer connector are each connected to the anesthesia machine or the ventilator through the breathing circuit; the laryngeal mask airway connector of the laryngeal mask component is connected to the patient end of the breathing circuit connector, and the inner tube body is inserted through the machine end second inner connector; as the inner tube body is gradually inserted, the apparatus dead space in the airway management system gradually decrease; when the patient end of the inner tube component is located in the mask body of the laryngeal mask component, and is aligned with the patient end of the laryngeal mask airway tube, the apparatus dead space can be eliminated; at this time, the patient end of the inner tube component passes through the glottis of the patient and enters the trachea of the patient, thereby eliminating all the apparatus dead space and some of the anatomic dead space. This method allows for the dead space volume of the airway management system to be continuously adjustable from being greater than that of the existing product to eliminating all the apparatus dead space plus some of the anatomic dead space, and is suitable for treating patients with hyperventilation.

In one embodiment, a first gas sampling cavity, and a first throat aspiration cavity are further provided in the laryngeal mask airway tube.

In one embodiment, a drainage cavity is further provided in the laryngeal mask airway tube.

In one embodiment, a video component cavity is further provided in the laryngeal mask airway tube.

In one embodiment, a standby reserved cavity is further provided in the laryngeal mask airway tube.

In one embodiment, the patient end of the first gas sampling cavity and the patient end of the first throat aspiration cavity are close to the patient end of the laryngeal mask airway tube, the first gas sampling cavity inside the laryngeal mask airway tube and the first throat aspiration cavity inside the laryngeal mask airway tube are fixedly mated.

The patient end of the first gas sampling cavity of the laryngeal mask component and the first gas sampling cavity are located on the ventral side of the patient. The patient end of the first gas sampling cavity is close to the patient end of the catheter, which can improve the sensitivity and accuracy of gas sampling analysis in the airway of the patient. The patient end of the first throat aspiration cavity of the laryngeal mask component and the throat aspiration cavity are located on the dorsal side of the patient; the patient end of the first throat aspiration cavity is close to the patient end of the laryngeal mask airway tube; and the first throat aspiration cavity can aspirate secretions near the glottis of the patient and or may serve as a standby gas sampling cavity. The patient end of the first gas sampling cavity, the first gas sampling cavity and the first throat aspiration cavity are fixedly mated, and are symmetrically provided, which guarantees the continuity and reliability of gas sampling analysis.

The first gas sampling cavity of the laryngeal mask component can be provided in the tube of the laryngeal mask airway tube, or may be independent outside the laryngeal mask airway tube. The first throat aspiration cavity and the first gas sampling cavity are symmetrically positioned and have identical structures. The patient end of the first throat aspiration cavity and the patient end of the first gas sampling chamber are close to the patient end of the catheter, symmetrically positioned, and have identical structures. The first throat aspiration cavity can be used to suction pharyngeal secretions, and can also serve as a standby sampling cavity when the gas sampling cavity is blocked, ensuring the continuity and reliability of gas sampling analysis.

The purpose of the second aspect of this invention is to provide an airway management system to solve the technical problem of difficult elimination of apparatus dead space.

The airway management system provided in the second aspect of this invention comprises an endotracheal tube component and an inner tube component, wherein the inner tube component comprises an inner tube body, a third ventilation cavity is arranged inside the inner tube body. The endotracheal tube component comprises an endotracheal tube, a second ventilation cavity is arranged in the endotracheal tube. The second ventilation cavity is configured for inserting the inner tube component; after inserting the inner tube component, between the second ventilation cavity and the third ventilation cavity, one forms an inhalation cavity and another forms an exhalation cavity. The endotracheal tube has the same internal diameter. The patient end of the inner tube component is configured to be align with the patient end of the endotracheal tube. The patient end of the inner tube component is also configured to enter the trachea of the patient through the patient end of the endotracheal tube.

The beneficial effects brought by the airway management system of this invention are:
By inserting the endotracheal tube component into the inner tube component, the inner tube connector and the endotracheal tube connector jointly connect to the patient end of the breathing circuit connector, where the breathing circuit connector is connected to an anesthesia machine or a ventilator through a breathing circuit, which can eliminate apparatus dead space and avoid that the patient inhales his/her own exhaled exhaust gas.

Endotracheal tube components of different specifications can be used in combination with inner tube components of different specifications to meet the needs of patients with different glottal areas and different ventilator parameter settings.

The purpose of the third aspect of this invention is to provide an airway management system to solve the technical problem of difficult elimination of apparatus dead space.

The airway management system provided in the third aspect of this invention comprises an endotracheal tube component and an inner tube component, wherein the inner tube component comprises an inner tube body, a third ventilation cavity is arranged inside the inner tube body. The second ventilation cavity is configured for inserting the inner tube component; after inserting the inner tube component, between the second ventilation cavity and the third ventilation cavity, one forms an inhalation cavity and another forms an exhalation cavity. The internal diameter of the endotracheal tube is configured such that the internal diameter of the segment located below the glottis of a patient when in use is smaller than that of the segment located above the glottis of the patient when in use. The patient end of the inner tube component is configured to be located at the patient end of the large diameter portion of the endotracheal tube of the unequal diameter structure.

The beneficial effects brought by the airway management system of this invention are:

The internal diameter of the endotracheal tube in this invention could be configured such that the internal diameter of the segment located below the glottis of a patient when in use is smaller than that of the segment located above the glottis of the patient when in use. This can avoid injure to the patient's glottis and trachea, improve patient comfort and reduce complications.

In one embodiment, the ventilation cavity formed after the second ventilation cavity is inserted into the inner tube component and the third ventilation cavity, one forms an inhalation cavity and another forms an exhalation cavity; or
the portion of the second ventilation cavity inserted into the inner tube component and the third ventilation cavity, one forms an inhalation cavity, and another forms an exhalation cavity.

In one embodiment, the endotracheal tube component comprises an endotracheal tube connector. The inner tube component comprises an inner tube connector connected to the inner tube body. The airway management system further comprises a breathing circuit connector component, the breathing circuit connector component comprises a breathing circuit connector, one end of the breathing circuit connector is connected to the endotracheal tube connector or to the endotracheal tube connector and the inner tube connector, the other end of the breathing circuit connector is configured to be connected to the anesthesia machine or ventilator via breathing circuit.

The breathing circuit connector comprises a patient end and a machine end of the breathing circuit connector. The patient end of the breathing circuit connector comprises a patient end inner connector and a patient end outer connector. The patient end outer connector is configured to be connected to the endotracheal tube connector, the patient end inner connector is configured to be connected to the inner tube connector.

The endotracheal tube, the endotracheal tube connector of this invention have internal diameters larger than that of the existing product, a diagnosis and treatment device with a larger external diameter can be implanted. A sampling site of the endotracheal tube component is close to the patient end of the catheter, which can make the sampling site close to the alveoli to the greatest extent, such that the sampling analysis is more sensitive and accurate, and a sampling analysis numerical value and an arterial blood gas analysis numerical value are more approximate and have good correlation.

In one embodiment, the machine end of the breathing circuit connector comprises a machine end first inner connector, a machine end second inner connector, and a machine end outer connector. The machine end of the breathing circuit connector is provided with three channels, the three channels being a machine end first inner channel, a machine end second inner channel, and a machine end outer channel. The patient end of the breathing circuit connector comprises a patient end inner channel, and a patient end outer channel. The machine end first inner channel and the machine end second inner channel is connected to the patient end inner channel, the machine end outer channel is connected to the patient end outer channel. The patient end inner channel and the patient end outer channel are of a coaxial inner and outer dual-channel structure. The machine end first inner connector and the machine end outer connector or the machine end second inner connector and the machine end outer connector are connected to the anesthesia machine or the ventilator through the breathing circuit, respectively.

After the laryngeal mask airway connector or the endotracheal tube connector is connected to the patient end of the breathing circuit connector component, the breathing circuit connector component can rotated in any direction within a circular range, facilitating the insertion and removal of the laryngeal mask airway connector of the laryngeal mask component or the endotracheal tube connector of the endotracheal tube component with the patient end of the breathing circuit connector component, and facilitating the placement of the breathing circuit.

The breathing circuit connector component employs a dual-channel design at the patient end and a three-channel design at the machine end, which can guarantee that the laryngeal mask component or the endotracheal tube component is implanted into the patient. After the laryngeal mask airway connector of the laryngeal mask component or the endotracheal tube connector of the laryngeal mask component is connected to the patient end of the breathing circuit connector component, the inner tube component is implanted under the guidance of a fiberoptic bronchoscope via the machine end second inner connector of the breathing circuit connector without interrupting low dead space volume ventilation. The patient end of the first Luer taper of the breathing circuit connector component of this invention can be connected to the gas-sampling line, and the gas-sampling line can collect gas inside the laryngeal mask airway tube, the endotracheal tube, on the glottis or inside the trachea for detection.

In one embodiment, the breathing circuit connector component further comprises a breathing circuit connector top end cover. The openable breathing circuit connector top end cover is provided at the top end of the machine end second inner connector. The breathing circuit connector top end cover is configured to be connected to the machine end first inner connector.

The laryngeal mask airway connector is connected to the patient end of the breathing circuit connector, or the endotracheal tube connector of the endotracheal tube component in which internal diameters are equal is connected to the patient end of the breathing circuit connector, and at this time, the breathing circuit connector top end cover is placed on the machine end first inner connector.

The endotracheal tube component can be connected to the patient end of the breathing circuit connector, and at this time, the breathing circuit connector top end cover is placed on the machine end first inner connector. By inserting the inner tube body into the machine end second inner connector, so that the inner tube connector and the machine end outer connector are each connected to the patient end of the breathing circuit, and the machine end of the breathing circuit is connected to the anesthesia machine or the ventilator. The endotracheal tube connector of the endotracheal tube component in which internal diameters are equal is connected to the patient end of the breathing circuit connector and after the machine end second inner connector of the breathing circuit connector is inserted into the inner tube body, as the inner tube body is gradually inserted, the apparatus dead space in the airway management system gradually decrease, when the patient end of the inner tube component and the patient end of the endotracheal tube are aligned, apparatus dead space is eliminated; the patient end of the inner tube component is further inserted and enters the trachea of the patient, thereby eliminating all the apparatus dead space and some of the anatomic dead space.

The endotracheal tube connector of the endotracheal tube component of a structure in which internal diameters are unequal is connected to the patient end of the breathing circuit connector, and the machine end second inner connector is inserted into the inner tube body; as the inner tube body is gradually inserted, the apparatus dead space in the airway management system gradually decrease; and when the patient end of the inner tube component is located at the patient end of a large internal diameter portion of the endotracheal tube of the unequal diameter structure, some of the apparatus dead space can be eliminated.

In one embodiment, the breathing circuit connector component further comprises a first Luer taper; and the middle of the breathing circuit connector top end cover is connected to the first Luer taper.

In one embodiment, the airway management system further comprises a connecting tube, and the connecting tube is of an unequal diameter structure. The connecting tube comprises a connecting tube body. When clinical anesthesia induction is performed, one end of the connecting tube body is configured to be connected to the patient end of the breathing circuit connector, and the other end of the connecting tube body is configured to be connected to the anesthetic mask.

In one embodiment, the connecting tube further comprises a second Luer taper. The second Luer taper is configured to be connected to the gas-sampling line of the monitor.

The connecting tube of this application is designed as the unequal diameter structure, and the second Luer taper of the connecting tube can serve as a gas sampling site during anesthesia induction and anesthesia maintenance. The patient end of the connecting tube can be connected to an existing mask, an existing laryngeal mask, the existing endotracheal tube, the laryngeal mask component of this application, the endotracheal tube component of this application, the laryngeal mask component and the inner tube component of this application, and the endotracheal tube component and the inner tube component of this application, and the machine end of the connecting tube is connected to the patient end of the breathing circuit connector component. After connection, the apparatus dead space can be enlarged, which is suitable for the induction stage of general anesthesia and the treatment of patients with hyperventilation.

In one embodiment, an outer wall of the inner tube body is provided with a plurality of protrusions; the protrusion is configured for positioning between the inner tube body and the first ventilation cavity or the second ventilation cavity.

There are several protrusions symmetrically designed in the axial direction on the outer wall of the inner tube body of the inner tube component in this invention, and after inserting into the ventilation cavity of the laryngeal mask airway tube or endotracheal tube, the inner tube body can be kept at the axis center position of the ventilation cavity into which the inner tube body is inserted under the support of the protrusions, which guarantees that the lumen of the ventilation cavity between the inner tube body and the laryngeal mask airway tube or the endotracheal tube is unimpeded. The inner tube component is of a detachable structure, and inner tube components of different internal diameters, external diameters, and lengths can be selected according to the anatomical characteristics of the throat and trachea of the patient and clinical use needs.

In one embodiment, a second throat aspiration cavity, a second gas sampling cavity, and a cuff inflation cavity are provided in the endotracheal tube.

In one embodiment, the patient end of the second gas sampling cavity and the patient end of the second throat aspiration cavity are close to the patient end of the endotracheal tube. The second gas sampling cavity and the second throat aspiration cavity are fixedly mated.

The patient end of the second gas sampling cavity of the endotracheal tube component and the gas sampling cavity are located on the ventral side of the patient. The patient end of the second gas sampling cavity is close to the patient end of the catheter, which can improve the sensitivity and accuracy of gas sampling analysis in the airway of the patient. The patient end of the second throat aspiration cavity of the endotracheal tube component and the throat aspiration cavity are located on the dorsal side of the patient; the patient end of the second throat aspiration cavity is close to the patient end of the catheter; and the second throat aspiration cavity can aspirate secretions near the glottis of the patient and or may serve as a standby gas sampling cavity. The patient end of the second gas sampling cavity, the patient end of the second gas sampling cavity and of the second throat aspiration cavity, the second throat aspiration cavity are fixedly mated, and are symmetrically provided, which guarantees the continuity and reliability of gas sampling analysis.

The second gas sampling cavity of the endotracheal tube component of this invention can be provided in the tube of the endotracheal tube, or may be independent outside the endotracheal tube, thus facilitating the design, research and development, and production of products.

### BRIEF DESCRIPTION OF THE DRAWINGS

The above and other objects, features and advantages of the exemplary embodiments of this invention will become readily understood by reading the following detailed description with reference to the accompanying drawings. In the accompanying drawings, several embodiments of this invention are illustrated in an exemplary rather than restrictive manner.

In the accompanying drawings, the same or corresponding reference numerals represent the same or corresponding parts.
FIG. 1 is a structural schematic diagram of embodiment 1 of this application;
FIG. 2 is a top view of FIG. 1; and
FIG. 2a is a cross-sectional schematic diagram along line A-A in FIG. 2;
FIG. 3 is a structural schematic diagram of a breathing circuit connector component of this application;
FIG. 4 is a top view of FIG. 3; and
FIG. 5 is a cross-sectional schematic diagram along line B-B in FIG. 3;
FIG. 6 is a cross-sectional schematic diagram along line C-C in FIG. 4;
FIG. 7 is a cross-sectional schematic diagram along line D-D in FIG. 6;
FIG. 8 is a structural schematic diagram of an inner tube component of this application;
FIG. 8a is a cross-sectional schematic diagram along line E-E in FIG. 8;
FIG. 9 is a schematic diagram of a telescopic tube component in an original state of this application;
FIG. 10 is a structural schematic diagram of a telescopic tube component in a fully extended state of this application;
FIG. 10a is a structural schematic diagram of a stopper of this application;
FIG. 11 is an assembly schematic diagram of a laryngeal mask component, an inner tube component and a telescopic tube component in an original state of this application;
FIG. 12 is an assembly schematic diagram of a laryngeal mask component, an inner tube component and a telescopic tube component in a fully extended state of this application;
FIG. 13 is a connection assembly schematic diagram of a laryngeal mask component and a breathing circuit connector component of this application;
FIG. 14 is an assembly schematic diagram of a laryngeal mask component and an inner tube component of this application;
FIG. 15 is an assembly schematic diagram of a laryngeal mask component, an inner tube component and a breathing circuit connector component of this application;
FIG. 16 is an assembly schematic diagram of a laryngeal mask component, and an inner tube component, a breathing circuit connector component, and a telescopic tube component of this application;
FIG. 17 is a structural schematic diagram of a connecting tube of this application;
FIG. 17a is a top view of FIG. 17;
FIG. 18 is a structural schematic diagram of embodiment 2 of this application;
FIG. 18a is a cross-sectional schematic diagram along line F-F in FIG. 18;
FIG. 19 is a structural schematic diagram of embodiment 3 of this application;
FIG. 19a is a cross-sectional schematic diagram along line G-G in FIG. 19;
FIG. 20 is an assembly schematic diagram of an endotracheal tube component and an inner tube component of embodiment 4 of this application;
FIG. 20a is a cross-sectional schematic diagram along line H-H in FIG. 20;
FIG. 20b is a cross-sectional schematic diagram along line I-I in FIG. 20;
FIG. 20c is an M-direction view in FIG. 20;
FIG. 21 is a top view of FIG. 20; and
FIG. 22 is an assembly schematic diagram of an endotracheal tube component, an inner tube component and a breathing circuit connector component of embodiment 4 of this application;
FIG. 23 is an assembly schematic diagram of an endotracheal tube component and an inner tube component of embodiment 5 of this application;
FIG. 23a is a cross-sectional schematic diagram along line J-J in FIG. 23;
FIG. 23b is a cross-sectional schematic diagram along line K-K in FIG. 23;
FIG. 23c is a cross-sectional schematic diagram along line L-L in FIG. 23;
FIG. 24 is a top view of FIG. 23; and
FIG. 25 is an assembly schematic diagram of an endotracheal tube component, an inner tube component and a breathing circuit connector component of embodiment 5 of this application.

### Reference numerals:

1. Laryngeal mask component; 1-1. Mask body; 1-2. laryngeal mask airway tube; 1-3. Laryngeal mask connector; 1-4. Laryngeal mask inflation tube; 1-5. Laryngeal mask inflation valve; 1-6. First ventilation cavity; 1-7. First throat aspiration cavity; 1-8. First gas sampling cavity; 1-9. Video component cavity; 1-10. Drainage cavity; 1-11. Standby reserved cavity; 1-12. First gas sampling line; 1-13. First throat aspiration tube; and 1-14. Third Luer taper;
2. endotracheal tube component; 2-1. cuff; 2-2. endotracheal tube; 2-3. endotracheal tube connector; 2-4. cuff inflation tube; 2-5. cuff inflation valve; 2-6. Second ventilation cavity; 2-7. Second throat aspiration cavity; 2-8. Second gas sampling cavity; 2-9. Second gas sampling line; 2-10. Second throat aspiration tube; 2-11. Fourth Luer taper; and 2-12. cuff inflation cavity;
3. breathing circuit connector component; 3-1. breathing circuit connector; 3-1-1. Patient end inner channel; 3-1-2. Patient end outer channel; 3-1-3. Machine end first inner channel; 3-1-4. Machine end second inner channel; 3-1-5. Machine end outer channel; 3-1-6. Patient end inner connector; 3-1-7. Patient end outer connector; 3-1-8. Machine end first inner connector; 3-1-9. Machine end second inner connector; 3-1-10. Machine end outer connector; 3-2. breathing circuit connector top end cover; and 3-3. First Luer taper;
4. Inner tube component; 4-1. Inner tube body; 4-2. Third ventilation cavity; 4-3. Inner tube connector; and 4-4. protrusion;
5. Connecting tube; 5-1. Connecting tube body; 5-2. Second Luer taper;
6. Telescopic tube component; 6-1. Telescopic tube body; 6-2. Telescopic tube patient end connector; 6-3. Telescopic tube machine end connector; 6-4. Stopper; and 6-5. Annular protrusion.

### DETAILED DESCRIPTION

In order to make the objects, technical solutions and advantages of this application more clearly understood, this application is further described in detail below in conjunction with the embodiments and the accompanying drawings. The described embodiments are merely some rather than all of the embodiments of this application. All other embodiments obtained by those skilled in the art based on the embodiments in this application without making creative efforts fall within the scope of protection of this application. It should be understood that the specific embodiments described here are only intended to explain this application and are not intended to limit this application.

In the descriptions of this specification, descriptions of reference terms such as "one embodiment", "some embodiments", "an embodiment", "a specific embodiment" or "some embodiments" means that specific features, structures, materials, or characteristics described with reference to the embodiment or the embodiment are comprised in at least one embodiment or embodiment of this application. Moreover, the described specific features, structures, materials, or characteristics can be combined in a proper manner in any one or more embodiments or embodiments. Furthermore, without contradictory, those skilled in the art can integrate and combine different embodiments or embodiments described in this specification and the features of different embodiments or embodiments.

Furthermore, the terms "first", "second", "third", and "fourth" are only used for descriptive purposes and cannot be understood as indicating or implying relative importance or implying the number of technical features indicated. Thus, the features defined as "first", "second", "third", and "fourth" may comprise at least one of these features explicitly or implicitly. In the description of this application, "a plurality of" means two or more, unless otherwise explicitly and specifically defined.

### Embodiment 1

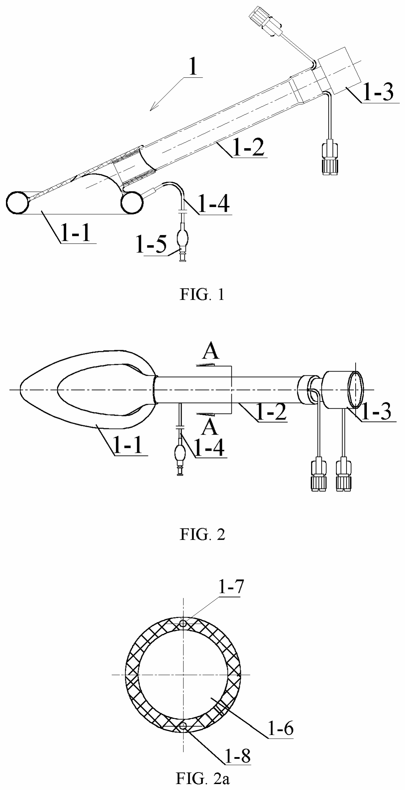

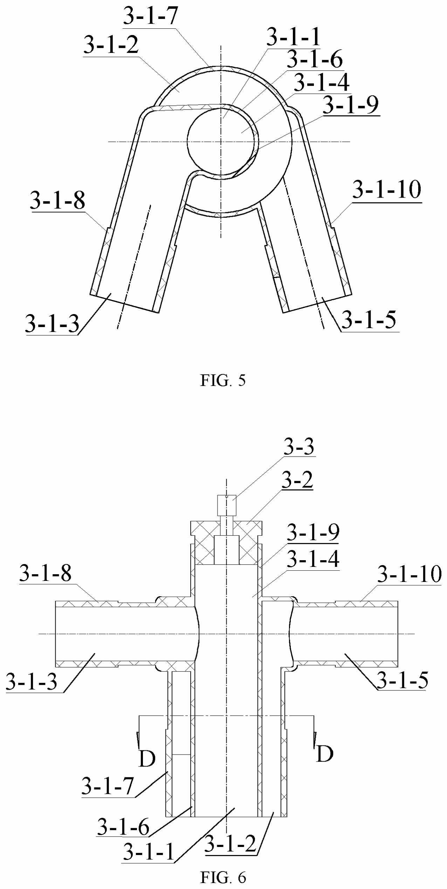

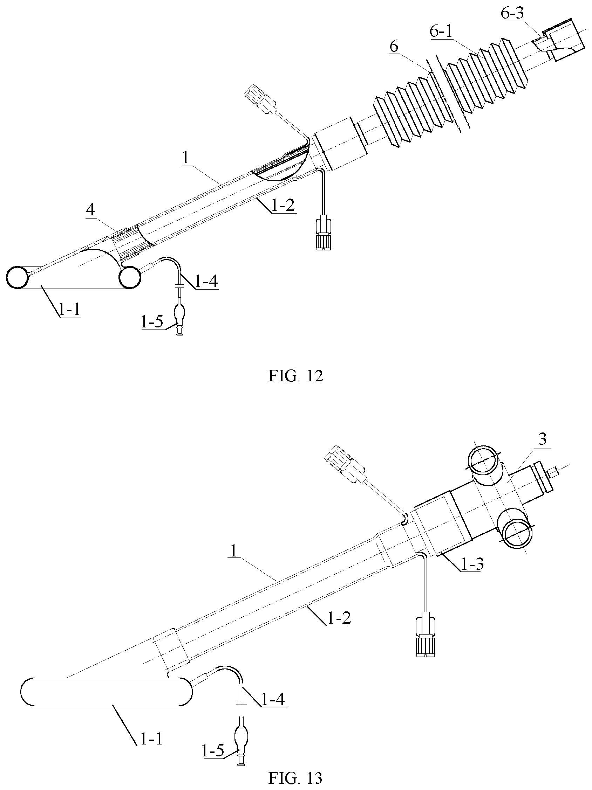

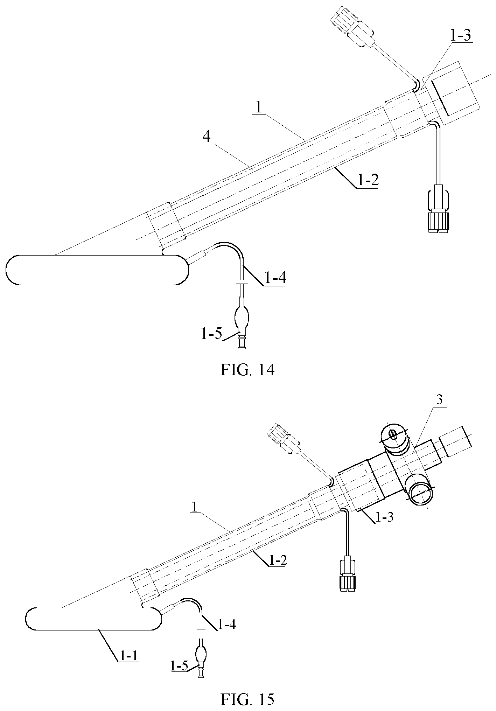

Referring to FIGS. 1 to 17a, an airway management system as shown comprises a laryngeal mask component 1, a breathing circuit connector component 3, an inner tube component 4, a connecting tube 5, and a telescopic tube component 6.

As shown in FIGS. 1 to 2, the laryngeal mask component 1 comprises a mask body 1-1, a laryngeal mask airway tube 1-2, a laryngeal mask connector 1-3, a laryngeal mask inflation tube 1-4, and a laryngeal mask inflation valve 1-5. One end of the laryngeal mask airway tube 1-2 is communicated with the mask body 1-1, and the other end of the laryngeal mask airway tube is communicated with the laryngeal mask connector 1-3. The laryngeal mask inflation tube 1-4 is provided on the exterior of the laryngeal mask airway tube 1-2. One end of the laryngeal mask inflation tube 1-4 is communicated with the mask body 1-1 to inflate the mask body, and the other end of the laryngeal mask inflation tube 1-4 is connected to the laryngeal mask inflation valve 1-5. As shown in FIG. 2a, three cavities are provided in the laryngeal mask airway tube 1-2, where the three cavities are a first ventilation cavity 1-6, a first gas sampling cavity 1-8, and a first throat aspiration cavity 1-7, respectively, where the first ventilation cavity 1-6 is located on a middle portion of the laryngeal mask airway tube 1-2 to facilitate insertion of the inner tube component 4. The first gas sampling cavity 1-8 and the first throat aspiration cavity 1-7 are each placed in the laryngeal mask airway tube 1-2, and a patient end of the first gas sampling cavity 1-8 and a patient end of the first throat aspiration cavity 1-7 are close to a patient end of the laryngeal mask airway tube 1-2. The first gas sampling cavity 1-8 is communicated with a first gas sampling line 1-12, the first throat aspiration cavity 1-7 is communicated with a first throat aspiration tube 1-13, and the other end of the first gas sampling line 1-12 and the other end of the first throat aspiration tube 1-13 are each connected to a third Luer taper 1-14. The third Luer taper 1-14 is connected to a monitor for analyzing the composition and concentration of the inhaled or exhaled gas of a patient. The first throat aspiration tube 1-13 may serve as an aspiration channel for secretions from the throat of the patient or may serve as a standby gas sampling line.

An internal diameter that can be employed for the laryngeal mask airway tube 1-2 of the laryngeal mask component 1 ranges from 3.3 to 25 mm. In some embodiments, the internal diameter of the laryngeal mask airway tube 1-2 may be any integer between 4 mm and 25 mm, or may also be 3.5 mm, 4.5 mm, 5.5 mm, 6.5 mm, 7.5 mm, 8.5 mm, 9.5 mm, 10.5 mm, 11.5 mm, 12.5 mm, 13.5 mm, 14.5 mm, 15.5 mm, 16.5 mm, 17.5 mm, 18.5 mm, 19.5 mm, 20.5 mm, 21.5 mm, 22.5 mm, 23.5 mm, 24.5 mm and so on.. An internal diameter that can be employed for the inner tube body 4-1 of the inner tube component 4 ranges from 2 to 12 mm. In some embodiments, the internal diameter of the inner tube body 4-1 may be any integer between 2 mm and 12 mm, or may also be 2.5 mm, 3.5 mm, 4.5 mm, 5.5 mm, 6.5 mm, 7.5 mm, 8.5 mm, 9.5 mm, 10.5 mm, 11.5 mm and so on..

In addition, the mask body 1-1 of the laryngeal mask component 1 can adopt the following specifications: 1#, 1.5#, 2#, 2.5#, 3#, 4#, 5#, 6#, etc. These specifications and dimensions are common sizes of existing products on the market, which will not be elaborated here.

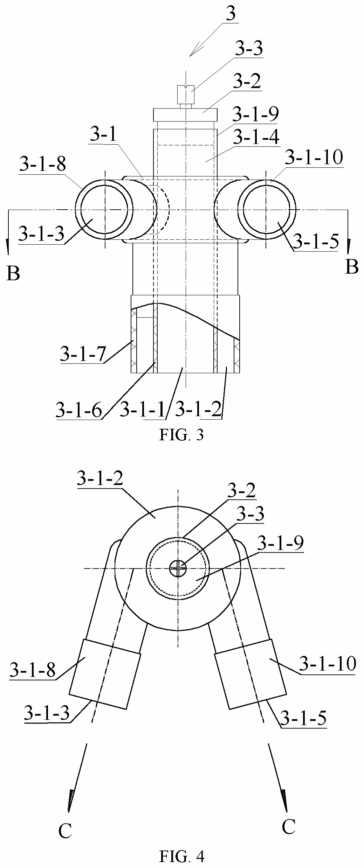

As shown in Figures 3-7, the breathing circuit connector component 3 comprises a breathing circuit connector 3-1, a breathing circuit connector top end cover 3-2, and a first Luer taper 3-3. The breathing circuit connector 3-1 comprises the patient end of the breathing circuit connector 3-1 and the machine end of the breathing circuit connector 3-1. The patient end of the breathing circuit connector 3-1 comprises a patient end inner connector 3-1-6 and a patient end outer connector 3-1-7. The machine end of the breathing circuit connector 3-1 comprises a machine end first inner connector 3-1-8, a machine end second inner connector 3-1-9, and a machine end outer connector 3-1-10; The patient end of the breathing circuit connector 3-1 is equipped with a patient end inner channel 3-1-1 and a patient end outer channel 3-1-2, where the patient end inner channel 3-1-1 and patient end outer channel 3-1-2 adopt a coaxial inner and outer dual-channel structure. The machine end of the breathing circuit connector 3-1 contains three channels, namely the machine end first inner channel 3-1-3 located within the machine end first inner connector 3-1-8, the machine end second inner channel 3-1-4 within the machine end second inner connector 3-1-9, and the machine end outer channel 3-1-5 within the machine end outer connector 3-1-10; The machine end first inner channel 3-1-3 of the breathing circuit connector 3-1, the machine end second inner channel 3-1-4 of the breathing circuit connector 3-1, and the patient end inner channel 3-1-1 of the breathing circuit connector 3-1 are connected. The machine end outer channel 3-1-5 of the breathing circuit connector 3-1 and the patient end outer channel 3-1-2 of the breathing circuit connector 3-1 are connected. An openable breathing circuit connector top end cover 3-2 is installed at the top of the machine end second inner connector 3-1-9 of the breathing circuit connector 3-1. The first Luer taper 3-3 is connected to the center of the breathing circuit connector top end cover 3-2. During clinical use, the patient end outer connector 3-1-7 of the breathing circuit connector 3-1 can connect to the laryngeal mask connector 1-3 or the telescopic tube patient end connector 6-2, while the patient end inner connector 3-1-6 of the breathing circuit connector 3-1 connects to the inner tube connector 4-3 of the inner tube component 4. The machine end first inner connector 3-1-8 and the machine end outer connector 3-1-10 of the breathing circuit connector 3-1 connect to the breathing circuit, which connects to the anesthesia machine or ventilator. When the breathing circuit connector top end cover 3-2 is removed, devices such as a fiberoptic bronchoscope, closed suction tube, or bronchial occluder can be inserted through the machine end second inner channel 3-1-4 of the breathing circuit connector 3-1; Alternatively, the machine end second inner connector 3-1-9 of the breathing circuit connector 3-1 and the machine end outer connector 3-1-10 of the breathing circuit connector 3-1 can be connected to the breathing circuit. Remove the breathing circuit connector top end cover 3-2 and place it at the machine end first inner connector 3-1-8 of the breathing circuit connector 3-1, with the breathing circuit connected to the anesthesia machine or ventilator.

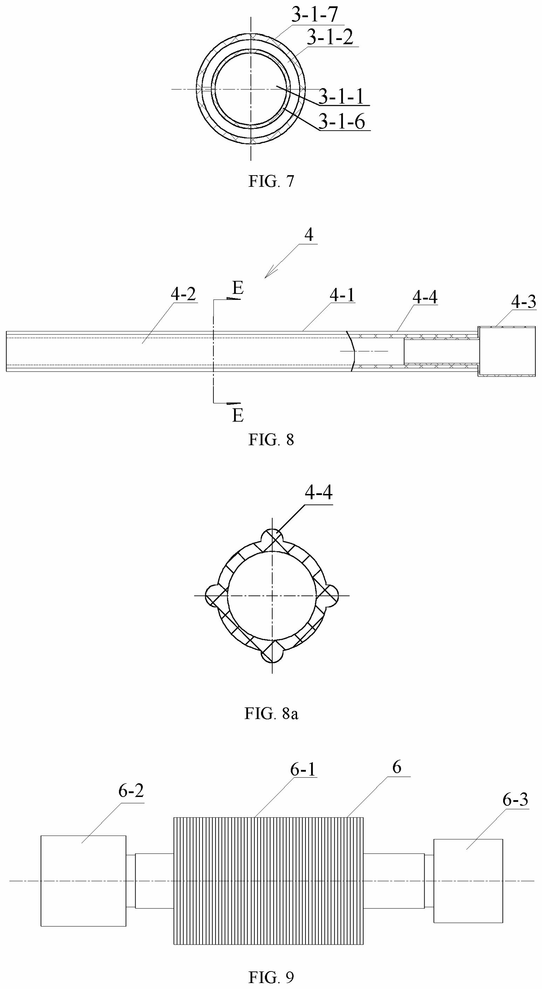

As shown in Figure 8-Figure 8a, the inner tube component 4 comprises an inner tube body 4-1 and an inner tube connector 4-3, with a third ventilation cavity 4-2 set inside the inner tube body 4-1. According to clinical needs, the inner tube component 4, closed suction tube, or bronchial occluder, etc. can be inserted into the first ventilation cavity 1-6 of the laryngeal mask component 1; After inserting the inner tube component 4, a closed suction tube or bronchial occluder can also be inserted into the inner tube body 4-1 of the inner tube component 4. The outer wall of the inner tube body 4-1 of the inner tube component 4 has multiple symmetrically arranged protrusions 4-4 along the axial direction, which are used for positioning the inner tube body 4-1 and the first ventilation cavity 1-6 inside the Laryngeal mask airway tube 1-2.

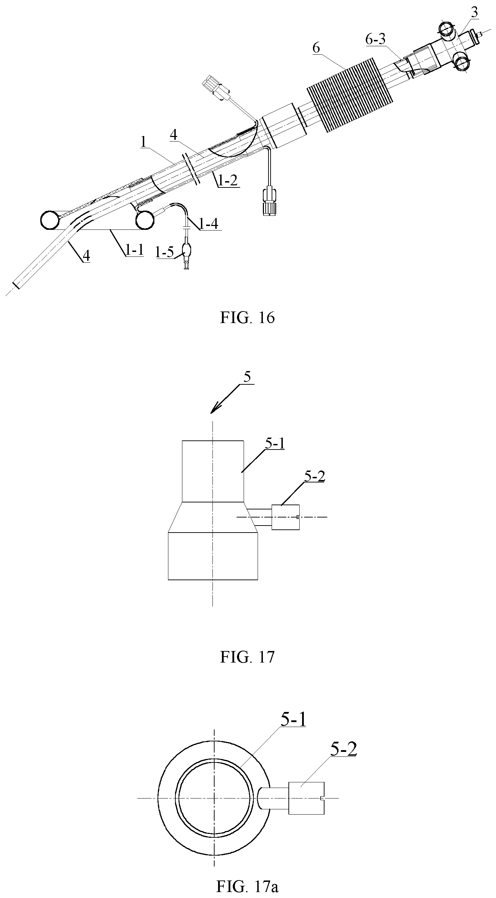

As shown in Figure 17-Figure 17a, the connecting tube 5 is of an unequal diameter structure. An equal diameter structure refers to a structure where the tube diameter (inner or external diameter) is completely identical or approximately identical throughout the connecting tube. The unequal diameter structure is opposite to the equal diameter structure. An unequal diameter structure refers to a structure where the tube diameter varies throughout the connecting tube, meaning the connecting tube has different inner (outer) diameter dimensions/sizes. Additionally, the connecting tube has the same wall thickness. The connecting tube 5 comprises a connecting tube body 5-1 and a second Luer taper 5-2. In clinical use, one end of the connecting tube body 5-1 connects to the patient end of the breathing circuit connector 3-1, while the other end connects to the anaesthetic mask. The second Luer taper 5-2 of the connecting tube 5 connects to the gas sampling line of the monitor, for use during anesthesia induction; One end of the connecting tube 5 can also connect to the connector of a standard endotracheal tube, with the other end connecting to the patient end of the breathing circuit connector 3-1. The second Luer taper 5-2 of the connecting tube 5 connects to the gas sampling line of the monitor, for use during intubation treatment via the laryngeal mask component 1.

As shown in Figures 9-16, the telescopic tube component 6 comprises a telescopic tube body 6-1, a telescopic tube patient end connector 6-2, and a telescopic tube machine end connector 6-3. The telescopic tube patient end connector 6-2 connects to the laryngeal mask connector 1-3 of the laryngeal mask component 1, and the telescopic tube machine end connector 6-3 connects to the patient end of the breathing circuit connector 3-1. The laryngeal mask connector 1-3 can connect to the telescopic tube patient end connector 6-2. When the telescopic tube component 6 is fully extended to its completely open state, the inner tube component 4 can be inserted through the laryngeal mask component 1 and the telescopic tube component 6. The patient end of the inner tube component 4 is positioned above the patient's glottis, inside the mask body 1-1 of the laryngeal mask component 1, and aligned with the patient end of the Laryngeal mask airway tube 1-2, which can eliminate the apparatus dead space. As the telescopic tube component 6 gradually contracts, the patient end of the inner tube component 4 moves toward the patient's lungs, passes through the patient's glottis, and enters the patient's trachea. When the telescopic tube component 6 contracts until it returns to its original state, the patient end of the inner tube component 4 reaches the patient's trachea, eliminating all apparatus dead space and part of the anatomic dead space.

During clinical implementation of anesthesia induction, the connecting tube 5 connects the patient end of the breathing circuit connector 3-1 to the anaesthetic mask, and the second Luer taper 5-2 of the connecting tube 5 connects to the gas sampling line of the monitor. The anaesthetic mask covers the patient's nose and mouth, and the breathing circuit connector 3-1 connects to the anesthesia machine or ventilator through the breathing circuit.

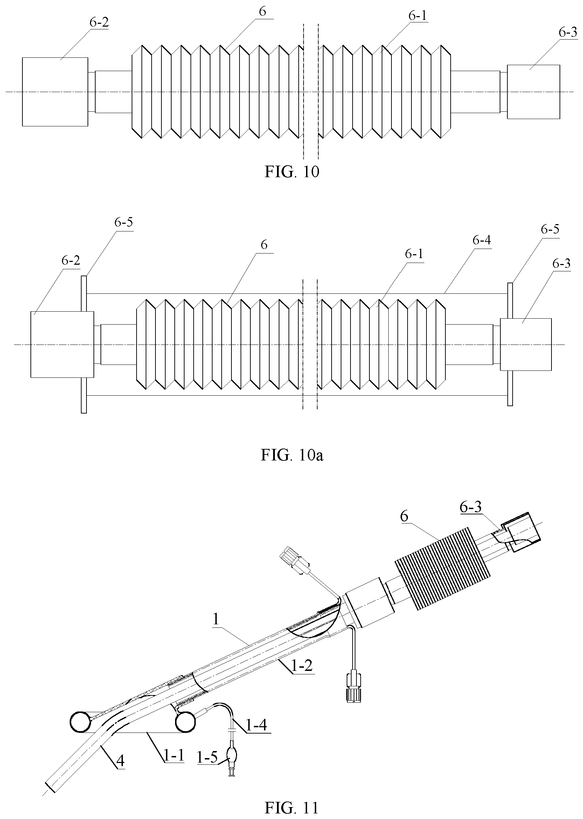

In one embodiment, as shown in Figure 10a, the telescopic tube component 6 of this application may also comprise: annular protrusions 6-5 and a stopper 6-4. The annular protrusions 6-5 are respectively set on the outer sidewalls of the telescopic tube patient end connector 6-2 and the telescopic tube machine end connector 6-3. The stopper 6-4 is used to limit the telescopic tube component 6. When the telescopic tube component 6 is fully extended to its completely open state, the stopper 6-4 is positioned between the annular protrusions 6-5 of the telescopic tube component 6. During use, when the telescopic tube component 6 is fully extended to its completely open state, the stopper 6-4 is placed between the annular protrusions 6-5 of the telescopic tube component 6. The inner tube component 4 can be inserted through the laryngeal mask component 1 and the telescopic tube component 6. The patient end of the inner tube component 4 is positioned above the patient's glottis, inside the mask body 1-1 of the laryngeal mask component 1, and aligned with the patient end of the Laryngeal mask airway tube 1-2. After removing the stopper 6-4, as the telescopic tube component 6 gradually contracts, the patient end of the inner tube component 4 moves toward the patient's lungs, passes through the patient's glottis, and enters the patient's trachea. The stopper 6-4 can be designed as a separable hollow cylindrical tube, as shown in Figure 10a, where the portion of the stopper 6-4 located above the telescopic tube body 6-1 can be the separable part. The stopper can be inserted or removed through the separable part of the stopper 6-4, allowing the telescopic tube component 6 to be positioned inside or outside the hollow cylindrical tube, thereby achieving the limiting or non-limiting function for the telescopic tube component 6.

The following will describe in detail the usage/working method of the airway management system in this application.

During anesthesia maintenance, after the patient is inserted with the Laryngeal mask component 1, connect the Laryngeal mask connector 1-3 of the Laryngeal mask component 1 to the patient end of the breathing circuit connector 3-1, and the breathing circuit connector 3-1 is connected to the anesthesia machine or ventilator through the breathing circuit. The third Luer taper 1-14 at the end of the first gas sampling line 1-12 of the laryngeal mask component 1 connects to the gas sampling line of the monitor to analyze the composition and concentration of gases inhaled or exhaled by the patient. The machine end inner connector 3-1-8 of the breathing circuit connector 3-1 and the machine end outer connector 3-1-10 of the breathing circuit connector 3-1 connect to the breathing circuit, or alternatively, the machine end second inner connector 3-1-9 of the breathing circuit connector 3-1 and the machine end outer connector 3-1-10 of the breathing circuit connector 3-1 connect to the breathing circuit, which connects to the anesthesia machine or ventilator. The machine end first inner connector 3-1-8 and machine end outer connector 3-1-10 of the breathing circuit connector 3-1 connect to the breathing circuit, when the breathing circuit connects to the anesthesia machine or ventilator, a fiberoptic bronchoscope, closed suction tube, bronchial occluder, etc. can be inserted through the machine end second inner channel 3-1-4 of the breathing circuit connector 3-1.

During anesthesia maintenance, after the patient is inserted with the laryngeal mask component 1, the inner tube component 4 can also be inserted through the Laryngeal mask airway tube 1-2 and laryngeal mask connector 1-3 of the laryngeal mask component 1, with the laryngeal mask connector 1-3 and inner tube connector 4-3 jointly connecting to the patient end of the breathing circuit connector 3-1. The patient end outer connector 3-1-7 of the breathing circuit connector 3-1 connects to the laryngeal mask connector 1-3, and the patient end inner connector 3-1-6 of the breathing circuit connector 3-1 connects to the inner tube connector 4-3 of the inner tube component 4; The machine end first inner connector 3-1-8 and machine end outer connector 3-1-10 of the breathing circuit connector 3-1 connect to the breathing circuit, alternatively the machine end second inner connector 3-1-9 and machine end outer connector 3-1-10 of the breathing circuit connector 3-1 can connect to the breathing circuit, which connects to the anesthesia machine or ventilator, eliminating apparatus dead space.

During anesthesia maintenance, after the patient is inserted with the laryngeal mask component 1, the laryngeal mask component 1 is connected to the patient end of the breathing circuit connector component 3, then the breathing circuit connector top end cover 3-2 is removed and placed on the machine end inner connector 3-1-8. The inner tube body 4-1 of the inner tube component 4 is inserted through the machine end second inner connector 3-1-9, the inner tube connector 4-3 of the inner tube component 4 and the machine end outer connector 3-1-10 of the breathing circuit connector 3-1 are connected to the patient end of the breathing circuit respectively, while the machine end of the breathing circuit is connected to the anesthesia machine or ventilator. The laryngeal mask component 1 connects to the patient end of the breathing circuit connector 3-1, and after the inner tube body 4-1 is inserted from the machine end second inner connector 3-1-9, the inner tube connector 4-3 of the inner tube component 4 and the machine end outer connector 3-1-10 of the breathing circuit connector 3-1 respectively connect to the patient end of the breathing circuit, while the machine end of the breathing circuit connects to the anesthesia machine or ventilator. As the inner tube body 4-1 is gradually inserted, the apparatus dead space within the airway management system gradually decreases; when the patient end of the inner tube component 4 is positioned inside the mask body 1-1 of the laryngeal mask component 1 and aligned with the patient end of the Laryngeal mask airway tube 1-2, the apparatus dead space is eliminated; The patient end of the inner tube component 4 can enter the trachea through the glottis, thereby eliminating all apparatus dead space and part of the anatomic dead space.

During anesthesia maintenance, after the patient is inserted with the laryngeal mask component 1, the laryngeal mask connector 1-3 can also be connected to the telescopic tube patient end connector 6-2. When the telescopic tube component 6 is fully extended to its completely open state, the inner tube component 4 can be inserted through the laryngeal mask component 1 and the telescopic tube component 6. At this time, the patient end of the inner tube component 4 is positioned above the patient's glottis, inside the mask body 1-1 of the laryngeal mask component 1, and aligned with the patient end of the Laryngeal mask airway tube 1-2. The patient end outer connector 3-1-7 of the breathing circuit connector 3-1 connects to the telescopic tube machine end connector 6-3, and the patient end inner connector 3-1-6 of the breathing circuit connector 3-1 connects to the inner tube connector 4-3 of the inner tube component 4; The machine end first inner connector 3-1-8 and machine end outer connector 3-1-10 of the breathing circuit connector 3-1 connect to the breathing circuit, or alternatively, the machine end second inner connector 3-1-9 and machine end outer connector 3-1-10 of the breathing circuit connector 3-1 connect to the breathing circuit, which connects to the anesthesia machine or ventilator, eliminating all apparatus dead space. As the telescopic tube component 6 gradually contracts, the patient end of the inner tube component 4 moves toward the patient's lungs, passing through the patient's glottis and entering the patient's trachea; When the telescopic tube component 6 contracts until it returns to its original state, the patient end of the inner tube component 4 can pass through the glottis into the trachea, thereby eliminating all apparatus dead space and part of the anatomic dead space. The third Luer taper 1-14 at the end of the first gas sampling line 1-12 of the laryngeal mask component 1 connects to the gas sampling line of the monitor to analyze the composition and concentration of gases inhaled or exhaled by the patient. In one example, when the telescopic tube component 6 is fully extended to its completely open state, stoppers 6-4 are placed between the annular protrusions 6-5 of the telescopic tube component 6, and before the telescopic tube component 6 contracts, the stoppers 6-4 are removed.

When inserting a regular endotracheal tube through the laryngeal mask component 1, the connector of the endotracheal tube connects to the patient end of the connecting tube 5, the machine end of the connecting tube 5 connects to the patient end of the breathing circuit connector 3-1, and the second Luer taper 5-2 of the connecting tube 5 connects to the gas sampling line of the monitor. When inserting other components of this application, such as the endotracheal tube component 2, through the laryngeal mask component 1, the inner tube component 4 is simultaneously inserted into the second ventilation cavity 2-6 of the endotracheal tube component 2. The endotracheal tube connector 2-3 of the endotracheal tube component 2 and the inner tube connector 4-3 of the inner tube component 4 jointly connect to the patient end of the breathing circuit connector 3-1. The fourth Luer taper 2-11 at the end of the second gas sampling line 2-9 of the endotracheal tube component 2 of this application connects to the gas sampling line of the monitor.

### Embodiment 2

Referring to Figures 3-18a, the airway management system shown in the figures comprises a laryngeal mask component 1, a breathing circuit connector component 3, an inner tube component 4, a connecting tube 5, and a telescopic tube component 6.

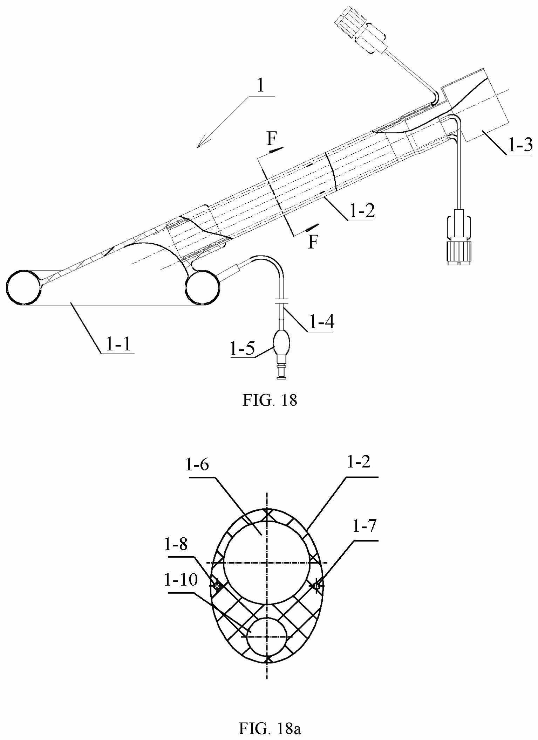

As shown in Figure 18, the laryngeal mask component 1 comprises a mask body 1-1, a Laryngeal mask airway tube 1-2, a laryngeal mask connector 1-3, a laryngeal mask inflation tube 1-4, and a laryngeal mask inflation valve 1-5. One end of the Laryngeal mask airway tube 1-2 is connected to the mask body 1-1, and the other end is connected to the laryngeal mask connector 1-3. The laryngeal mask inflation tube 1-4 is positioned on the exterior of the Laryngeal mask airway tube 1-2, with one end connected to the mask body 1-1 for inflation, and the other end of the laryngeal mask inflation tube 1-4 is connected to the laryngeal mask inflation valve 1-5. Different from embodiment 1, the Laryngeal mask airway tube 1-2 of this embodiment has four internal chambers, namely the first ventilation cavity 1-6 which can be used for inserting the inner tube body 4-1, the first gas sampling cavity 1-8, the first throat aspiration cavity 1-7, and the drainage cavity 1-10, as shown in Figure 18a. The first ventilation cavity 1-6, the first gas sampling cavity 1-8, the first throat aspiration cavity 1-7, and the drainage cavity 1-10 are placed inside the Laryngeal mask airway tube 1-2, with the patient end of the first gas sampling cavity 1-8 and the patient end of the first throat aspiration cavity 1-7 located near the patient end of the Laryngeal mask airway tube 1-2. The first gas sampling cavity 1-8 is connected to the first gas sampling line 1-12, and the first throat aspiration cavity 1-7 is connected to the first throat aspiration tube 1-13. The other ends of the first gas sampling line 1-12 and the first throat aspiration tube 1-13 are connected to a third Luer taper 1-14. The third Luer taper 1-14 connects to a monitor for analyzing the composition and concentration of gases inhaled or exhaled by the patient. The first throat aspiration tube 1-13 can serve as a suction channel for pharyngeal secretions from the patient, and can also function as a backup gas sampling line.

In this embodiment, the breathing circuit connector component 3, inner tube component 4, connecting tube 5, and telescopic tube component 6 have similar structures and functions to the breathing circuit connector component 3, inner tube component 4, connecting tube 5, and telescopic tube component 6 in the aforementioned embodiment 1, which will not be repeated here.

When using the airway management system in this embodiment, it comprises the usage/working methods mentioned in the previous embodiment 1, and the identical parts will not be repeated here. The difference from the usage/working method of embodiment 1 is: during anesthesia maintenance, after the patient is inserted with the laryngeal mask component 1, the patient end of the drainage cavity 1-10 of the laryngeal mask component 1 is connected to the patient's esophageal opening to drain the patient's gastric contents, and a gastric tube can be inserted along the drainage cavity 1-10.

### Embodiment 3

Please refer to Figure 3-Figure 17a, Figure 19-Figure 19a, the airway management system shown in the figures comprises a laryngeal mask component 1, a breathing circuit connector component 3, an inner tube component 4, a connecting tube 5, and a telescopic tube component 6.

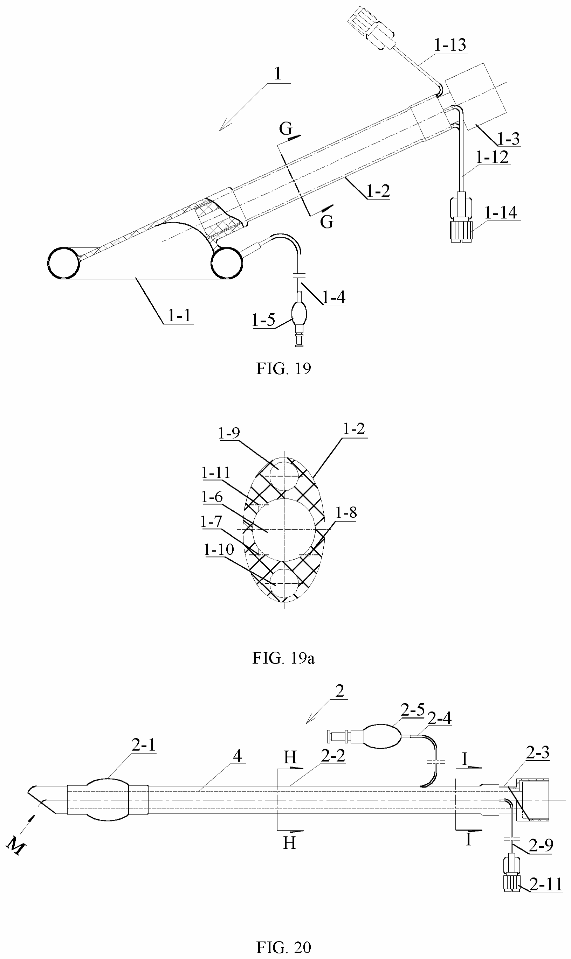

As shown in Figure 19, the laryngeal mask component 1 comprises a mask body 1-1, a Laryngeal mask airway tube 1-2, a laryngeal mask connector 1-3, a laryngeal mask inflation tube 1-4, and a laryngeal mask inflation valve 1-5. One end of the Laryngeal mask airway tube 1-2 is connected to the mask body 1-1, and the other end is connected to the laryngeal mask connector 1-3. The laryngeal mask inflation tube 1-4 is positioned on the exterior of the Laryngeal mask airway tube 1-2, with one end connected to the mask body 1-1 for inflation, and the other end of the laryngeal mask inflation tube 1-4 is connected to the laryngeal mask inflation valve 1-5. Different from Embodiments 1-2, the Laryngeal mask airway tube 1-2 of this embodiment has six internal chambers, namely a first ventilation cavity 1-6 for inserting the inner tube body 4-1, a first gas sampling cavity 1-8, a first throat aspiration cavity 1-7, a video component cavity 1-9, a drainage cavity 1-10, and a standby reserved cavity 1-11, as shown in Figure 19a. The first ventilation cavity 1-6 is located in the central part of the Laryngeal mask airway tube 1-2 to facilitate the insertion of the inner tube component 4. The first gas sampling cavity 1-8, first throat aspiration cavity 1-7, video component cavity 1-9, drainage cavity 1-10, and standby reserved cavity 1-11 are respectively placed inside the Laryngeal mask airway tube 1-2, with the patient end of the first gas sampling cavity 1-8 and the patient end of the first throat aspiration cavity 1-7 positioned close to the patient end of the Laryngeal mask airway tube 1-2. The first gas sampling cavity 1-8 is connected to the first gas sampling line 1-12, and the first throat aspiration cavity 1-7 is connected to the first throat aspiration tube 1-13. The other ends of the first gas sampling line 1-12 and the first throat aspiration tube 1-13 are connected to a third Luer taper 1-14. The third Luer taper 1-14 connects to a monitor for analyzing the composition and concentration of gases inhaled or exhaled by the patient. The first throat aspiration tube 1-13 can serve as a suction channel for pharyngeal secretions from the patient, and can also function as a backup gas sampling line.

The breathing circuit connector component 3, inner tube component 4, connecting tube 5, and telescopic tube component 6 in this embodiment have similar structures and functions to the breathing circuit connector component 3, inner tube component 4, connecting tube 5, and telescopic tube component 6 in the aforementioned embodiments 1-2, which will not be elaborated here.

When in use, the airway management system in this embodiment comprises the usage/working methods from the aforementioned Embodiment 2, and the identical parts will not be repeated. The difference from the usage/working method of Embodiment 2 is: during anesthesia maintenance, after the patient is inserted with the laryngeal mask component 1, a video device can be inserted through the video component cavity 1-9 of the laryngeal mask component 1 and connected to a display, which can be used for: (1) observing the position of the mask body 1-1 of the laryngeal mask component 1, the status of the glottis, and the condition of pharyngeal secretions, thereby making appropriate interventions. (2) When the patient end of the inner tube component 4 moves toward the patient's lungs, it can enter the trachea through the glottis under video monitoring guidance, avoiding injure to the glottis and trachea. (3) When inserting the endotracheal tube through the Laryngeal mask component 1, it can be performed under video monitoring guidance, avoiding glottis and trachea injury. The patient end of the video component cavity 1-9 of the laryngeal mask component 1 can be cleaned through the standby reserved cavity 1-11 of the laryngeal mask component 1, maintaining clarity and transparency at the patient end of the video component cavity 1-9.

### Embodiment 4

Please refer to Figure 3-Figure 8a, Figure 17-Figure 17a, Figure 20-Figure 22, which show an airway management system comprising an endotracheal tube component 2, a breathing circuit connector component 3, an inner tube component 4, and a connecting tube 5.

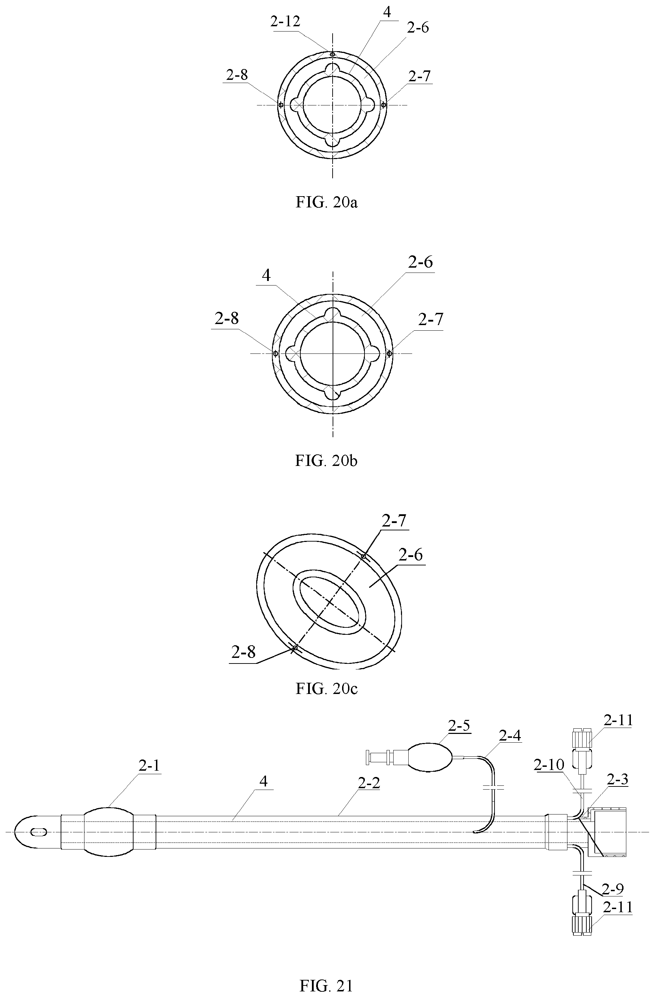

As shown in Figure 20-Figure 22, the endotracheal tube component 2 comprises a cuff 2-1, an endotracheal tube 2-2, an endotracheal tube connector 2-3, a cuff inflation tube 2-4, and a cuff inflation valve 2-5. The endotracheal tube 2-2 of the endotracheal tube component 2 has four cavities, namely the second ventilation cavity 2-6, the second throat aspiration cavity 2-7, the cuff inflation cavity 2-12, and the second gas sampling cavity 2-8. The internal diameter of the second ventilation cavity 2-6 and of the endotracheal tube 2-2 are equal in each section of the lumen or are designed to have the same internal diameter; in other words, the internal diameter/size of the second ventilation cavity 2-6 and the endotracheal tube 2-2 is the same at all points. The patient end of the second gas sampling cavity 2-8 and the patient end of the second throat aspiration cavity 2-7 are close to the patient end of the endotracheal tube 2-2. The machine end of the cuff inflation cavity 2-12 is connected to the cuff inflation tube 2-4, and the cuff inflation tube 2-4 is connected to the cuff inflation valve 2-5. The second ventilation cavity 2-6 of the endotracheal tube component 2 is located in the central part of the endotracheal tube 2-2, to facilitate the insertion of the inner tube component 4. The cuff inflation cavity 2-12, the second gas sampling cavity 2-8, and the second throat aspiration cavity 2-7 are placed inside the endotracheal tube 2-2. The second throat aspiration cavity 2-7 is connected to the second throat aspiration tube 2-10, the second gas sampling cavity 2-8 is connected to the second gas sampling line 2-9, and the machine ends of the second throat aspiration tube 2-10 and the second gas sampling line 2-9 are connected to the fourth Luer taper 2-11. The fourth Luer taper 2-11 connects to the monitor, used for analyzing the composition and concentration of gases inhaled or exhaled by the patient. The second throat aspiration tube 2-10 can serve as an aspiration channel for secretions from the throat of the patient, and can also function as a backup gas sampling line.

The endotracheal tube 2-2 of the endotracheal tube component 2 can have an internal diameter ranging from 2 to 20 millimeters. In some examples, the internal diameter of the endotracheal tube 2-2 can be any integer between 2 millimeters and 20 millimeters, and can also be: 2.5 millimeters, 3.5 millimeters, 4.5 millimeters, 5.5 millimeters, 6.5 millimeters, 7.5 millimeters, 8.5 millimeters, 9.5 millimeters, 10.5 millimeters, 11.5 millimeters, 12.5 millimeters, 13.5 millimeters, 14.5 millimeters, 15.5 millimeters, 16.5 millimeters, 17.5 millimeters, 18.5 millimeters, 19.5 millimeters, etc. The internal diameter range of the inner tube body 4-1 of the inner tube component 4 can be 2 to 12 millimeters. In some examples, the internal diameter of the inner tube body 4-1 can be any integer between 2 millimeters and 12 millimeters, and can also be: 2.5 millimeters, 3.5 millimeters, 4.5 millimeters, 5.5 millimeters, 6.5 millimeters, 7.5 millimeters, 8.5 millimeters, 9.5 millimeters, 10.5 millimeters, 11.5 millimeters, etc.

As shown in Figures 3-7, the breathing circuit connector component 3 comprises a breathing circuit connector 3-1, a breathing circuit connector top end cover 3-2, and a first Luer taper 3-3. The breathing circuit connector 3-1 comprises the patient end of the breathing circuit connector 3-1 and the machine end of the breathing circuit connector 3-1. The patient end of the breathing circuit connector 3-1 comprises a patient end inner connector 3-1-6 and a patient end outer connector 3-1-7. The machine end of the breathing circuit connector 3-1 comprises a machine end first inner connector 3-1-8, a machine end second inner connector 3-1-9, and a machine end outer connector 3-1-10; The patient end of the breathing circuit connector 3-1 is equipped with a patient end inner channel 3-1-1 and a patient end outer channel 3-1-2, where the patient end inner channel 3-1-1 and patient end outer channel 3-1-2 adopt a coaxial inner and outer dual-channel structure. The machine end of the breathing circuit connector 3-1 contains three channels, namely the machine end first inner channel 3-1-3 located within the machine end first inner connector 3-1-8, the machine end second inner channel 3-1-4 within the machine end second inner connector 3-1-9, and the machine end outer channel 3-1-5 within the machine end outer connector 3-1-10; The first inner channel 3-1-3 at the machine end of the breathing circuit connector 3-1, the second inner channel 3-1-4 at the machine end of the breathing circuit connector 3-1, and the inner channel 3-1-1 at the patient end of the breathing circuit connector 3-1 are connected. The outer channel 3-1-5 at the machine end of the breathing circuit connector 3-1 and the outer channel 3-1-2 at the patient end of the breathing circuit connector 3-1 are connected. An openable breathing circuit connector top end cover 3-2 is installed at the top of the second inner connector 3-1-9 at the machine end of the breathing circuit connector 3-1. The first Luer taper 3-3 is connected to the center of the breathing circuit connector top end cover 3-2. During clinical use, the patient end outer connector 3-1-7 of the breathing circuit connector 3-1 connects to the endotracheal tube connector 2-3, and the patient end inner connector 3-1-6 of the breathing circuit connector 3-1 connects to the inner tube connector 4-3 of the inner tube component 4. The machine end first inner connector 3-1-8 and the machine end outer connector 3-1-10 of the breathing circuit connector 3-1 connect to the breathing circuit, which connects to the anesthesia machine or ventilator. When the breathing circuit connector top end cover 3-2 is removed, devices such as a fiberoptic bronchoscope, closed suction tube, or bronchial occluder can be inserted through the machine end second inner channel 3-1-4 of the breathing circuit connector 3-1; Alternatively, the machine end second inner connector 3-1-9 of the breathing circuit connector 3-1 and the machine end outer connector 3-1-10 of the breathing circuit connector 3-1 can be connected to the breathing circuit. Remove the breathing circuit connector top end cover 3-2 and place it at the machine end first inner connector 3-1-8 of the breathing circuit connector 3-1, with the breathing circuit connected to the anesthesia machine or ventilator.

As shown in Figure 8-Figure 8a and Figure 21-Figure 22, the inner tube component 4 comprises an inner tube body 4-1 and an inner tube connector 4-3, with a third ventilation cavity 4-2 set inside the inner tube body 4-1. According to clinical needs, the inner tube component 4, closed suction tube, or bronchial occluder, etc., can be inserted into the second ventilation cavity 2-6 of the endotracheal tube component 2; After inserting the inner tube component 4, a closed suction tube or bronchial occluder can also be inserted into the inner tube body 4-1 of the inner tube component 4. The outer wall of the inner tube body 4-1 of the inner tube component 4 has multiple symmetrically arranged protrusions 4-4 along the axial direction, which are used for positioning the inner tube body 4-1 and the second ventilation cavity 2-6 inside the endotracheal tube 2-2.

As shown in Figure 17-Figure 17a, the connecting tube 5 has an unequal diameter structure, comprising a connecting tube body 5-1 and a second Luer taper 5-2. In clinical use, one end of the connecting tube body 5-1 connects to the patient end of the breathing circuit connector 3-1, and the other end connects to the anaesthetic mask. The second Luer taper 5-2 of the connecting tube 5 connects to the gas sampling line of the monitor, which is used for anesthesia induction.

During clinical implementation of anesthesia induction, the connecting tube 5 connects the patient end of the breathing circuit connector 3-1 to the anaesthetic mask, and the second Luer taper 5-2 of the connecting tube 5 connects to the gas sampling line of the monitor. The anaesthetic mask covers the patient's nose and mouth, and the breathing circuit connector 3-1 connects to the anesthesia machine or ventilator through the breathing circuit.

During anesthesia maintenance, after the patient is inserted with the endotracheal tube component 2, the endotracheal tube connector 2-3 connects to the patient end of the breathing circuit connector 3-1, and the machine end of the breathing circuit connector 3-1 connects to the anesthesia machine or ventilator through the breathing circuit. The fourth Luer taper 2-11 of the second gas sampling line 2-9 of the endotracheal tube component 2 connects to the monitor gas sampling line, analyzing the composition and concentration of gases inhaled or exhaled by the patient. The machine end first inner connector 3-1-8 and machine end outer connector 3-1-10 of the breathing circuit connector 3-1 connect to the breathing circuit, or alternatively, the machine end second inner connector 3-1-9 and machine end outer connector 3-1-10 of the breathing circuit connector 3-1 connect to the breathing circuit, which connects to the anesthesia machine or ventilator. The machine end first inner connector 3-1-8 and machine end outer connector 3-1-10 of the breathing circuit connector 3-1 connect to the breathing circuit, when the breathing circuit connects to the anesthesia machine or ventilator, a fiberoptic bronchoscope, closed suction tube, bronchial occluder, etc. can be inserted through the machine end second inner channel 3-1-4 of the breathing circuit connector 3-1.

During anesthesia maintenance, after the patient is inserted with the endotracheal tube component 2, an inner tube component 4 can also be inserted through the endotracheal tube 2-2 and endotracheal tube connector 2-3 of the endotracheal tube component 2. The patient end of the inner tube component 4 aligns with the patient end of the endotracheal tube 2-2, while the endotracheal tube connector 2-3 and the inner tube connector 4-3 jointly connect to the patient end of the breathing circuit connector 3-1. The patient end outer connector 3-1-7 of the breathing circuit connector 3-1 connects to the endotracheal tube connector 2-3, and the patient end inner connector 3-1-6 of the breathing circuit connector 3-1 connects to the inner tube connector 4-3 of the inner tube component 4. The machine end first inner connector 3-1-8 and machine end outer connector 3-1-10 of the breathing circuit connector 3-1 connect to the breathing circuit, or alternatively, the machine end second inner connector 3-1-9 and machine end outer connector 3-1-10 of the breathing circuit connector 3-1 connect to the breathing circuit. The breathing circuit connects to the anesthesia machine or ventilator, which can eliminate all apparatus dead space. The fourth Luer taper 2-11 at the end of the second gas sampling line 2-9 of the endotracheal tube component 2 connects to the monitor gas sampling line, analyzing the composition and concentration of gases inhaled or exhaled by the patient.

During anesthesia maintenance, after the patient is inserted with the endotracheal tube component 2, the endotracheal tube connector 2-3 of the endotracheal tube component 2 connects to the patient end of the breathing circuit connector component 3, then remove the breathing circuit connector top end cover 3-2 and place it on the machine end first inner connector 3-1-8. The inner tube body 4-1 of the inner tube component 4 is inserted through the machine end second inner connector 3-1-9 of the breathing circuit connector 3-1, the inner tube connector 4-3 of the inner tube component 4 and the machine end outer connector 3-1-10 of the breathing circuit connector 3-1 are connected to the patient end of the breathing circuit respectively, while the machine end of the breathing circuit connects to the anesthesia machine or ventilator. The endotracheal tube connector 2-3 of the endotracheal tube component 2 connects to the patient end of the breathing circuit connector 3-1, and after the inner tube body 4-1 is inserted through the second internal channel 3-1-4 of the machine end of the breathing circuit connector 3-1, the inner tube connector 4-3 of the inner tube component 4 and the machine end outer connector 3-1-10 of the breathing circuit connector 3-1 are respectively connected to the patient end of the breathing circuit, while the machine end of the breathing circuit connects to the anesthesia machine or ventilator; As the inner tube body 4-1 is gradually inserted, the apparatus dead space within the airway management system gradually decreases, and when the patient end of the inner tube component 4 aligns with the patient end of the endotracheal tube 2-2, all apparatus dead space is eliminated. The patient end of the inner tube component 4 can continue to be inserted into the trachea, thereby eliminating all apparatus dead space plus part of the anatomic dead space.

### Embodiment 5

Please refer to Figures 3-8a, Figures 17-17a, Figures 23-25, which show an airway management system, comprising an endotracheal tube component 2, a breathing circuit connector component 3, an inner tube component 4, and a connecting tube 5.

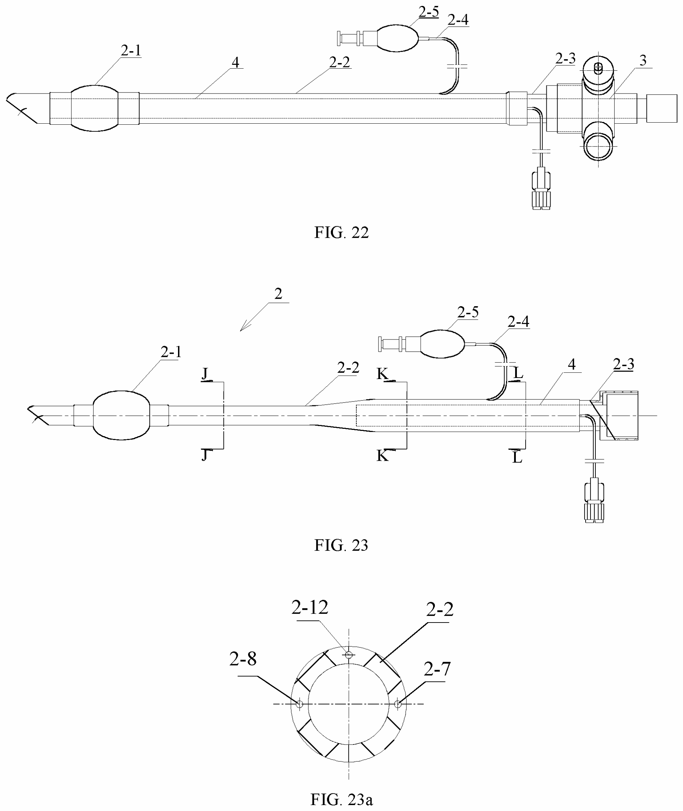

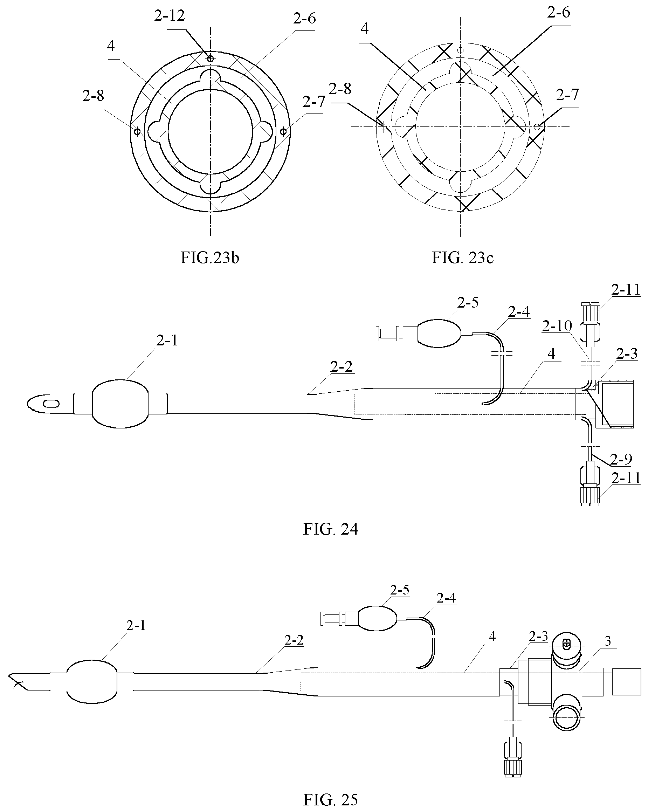

As shown in Figures 23-25, the endotracheal tube component 2 comprises a cuff 2-1, an endotracheal tube 2-2, an endotracheal tube connector 2-3, a cuff inflation tube 2-4, and a cuff inflation valve 2-5. The endotracheal tube component 2 has four cavities inside the endotracheal tube 2-2, namely the second ventilation cavity 2-6, the second throat aspiration cavity 2-7, the cuff inflation cavity 2-12, and the second gas sampling cavity 2-8. Different from Embodiment 4, in this embodiment, the second ventilation cavity 2-6 and the endotracheal tube 2-2 are designed with a structure where the internal diameter of the section below the patient's glottis is smaller than the internal diameter of the section above the patient's glottis. In other words, the internal diameters/dimensions of the second ventilation cavity 2-6 and the endotracheal tube 2-2 vary at different locations, with the internal diameter/dimension of the section positioned below the patient's glottis during use being smaller than the internal diameter/dimension of the section positioned above the patient's glottis during use. The patient end of the second gas sampling cavity 2-8 and the patient end of the second throat aspiration cavity 2-7 are close to the patient end of the endotracheal tube 2-2. The machine end of the cuff inflation cavity 2-12 is connected to the cuff inflation tube 2-4, and the cuff inflation tube 2-4 is connected to the cuff inflation valve 2-5. The second ventilation cavity 2-6 is located in the central part of the endotracheal tube 2-2, to facilitate the insertion of the inner tube component 4. The cuff inflation cavity 2-12, the second gas sampling cavity 2-8, and the second throat aspiration cavity 2-7 are placed inside the endotracheal tube 2-2. The second throat aspiration cavity 2-7 is connected to the second throat aspiration tube 2-10, the second gas sampling cavity 2-8 is connected to the second gas sampling line 2-9, and the machine ends of the second throat aspiration tube 2-10 and the second gas sampling line 2-9 are connected to the fourth Luer taper 2-11. The fourth Luer taper 2-11 connects to the monitor, used for analyzing the composition and concentration of gases inhaled and exhaled by the patient. The second throat aspiration tube 2-10 can serve as an aspiration channel for secretions from the throat of the patient, and can also function as a backup gas sampling line.

The breathing circuit connector component 3, inner tube component 4, and connecting tube 5 in this embodiment have similar structures and functions to the breathing circuit connector component 3, inner tube component 4, and connecting tube 5 in the aforementioned Embodiment 4, which will not be elaborated here.

During anesthesia maintenance, after the patient is inserted with the endotracheal tube component 2, the endotracheal tube connector 2-3 of the endotracheal tube component 2 is connected to the patient end of the breathing circuit connector 3-1, and the machine end of the breathing circuit connector 3-1 is connected to the anesthesia machine or ventilator via breathing circuit. The fourth Luer taper 2-11 of the second gas sampling line 2-9 of the endotracheal tube component 2 connects to the monitor gas sampling line, analyzing the composition and concentration of gases inhaled or exhaled by the patient. The machine end first inner connector 3-1-8 and machine end outer connector 3-1-10 of the breathing circuit connector 3-1 connect to the breathing circuit, or alternatively, the machine end second inner connector 3-1-9 and machine end outer connector 3-1-10 of the breathing circuit connector 3-1 connect to the breathing circuit, which connects to the anesthesia machine or ventilator. The machine end first inner connector 3-1-8 and machine end outer connector 3-1-10 of the breathing circuit connector 3-1 connect to the breathing circuit, when the breathing circuit connects to the anesthesia machine or ventilator, a fiberoptic bronchoscope, closed suction tube, bronchial occluder, etc. can be inserted through the machine end second inner channel 3-1-4 of the breathing circuit connector 3-1.

During anesthesia maintenance, after the patient is inserted with the endotracheal tube component 2, an inner tube component 4 can also be inserted through the endotracheal tube 2-2 and endotracheal tube connector 2-3 of the endotracheal tube component 2. The patient end of the inner tube component 4 is positioned at the patient end of the larger internal diameter portion of the endotracheal tube 2-2. The endotracheal tube connector 2-3 and the inner tube connector 4-3 jointly connect to the patient end of the breathing circuit connector 3-1. The patient end outer connector 3-1-7 of the breathing circuit connector 3-1 connects to the endotracheal tube connector 2-3, and the patient end inner connector 3-1-6 of the breathing circuit connector 3-1 connects to the inner tube connector 4-3 of the inner tube component 4; The machine end first inner connector 3-1-8 and machine end outer connector 3-1-10 of the breathing circuit connector 3-1 connect to the breathing circuit, or alternatively, the machine end second inner connector 3-1-9 and machine end outer connector 3-1-10 of the breathing circuit connector 3-1 connect to the breathing circuit. The breathing circuit connects to the anesthesia machine or ventilator, which can eliminate part of the apparatus dead space. The fourth Luer taper 2-11 at the end of the second gas sampling line 2-9 of the endotracheal tube component 2 connects to the monitor gas sampling line, analyzing the composition and concentration of gases inhaled or exhaled by the patient.

During anesthesia maintenance, after the patient is inserted with the endotracheal tube component 2, the endotracheal tube connector 2-3 of the endotracheal tube component 2 connects to the patient end of the breathing circuit connector component 3, then remove the breathing circuit connector top end cover 3-2 and place it on the machine end first inner connector 3-1-8. The inner tube body 4-1 of the inner tube component 4 is inserted through the machine end second inner connector 3-1-9 of the breathing circuit connector 3-1, the inner tube connector 4-3 of the inner tube component 4 and the machine end outer connector 3-1-10 of the breathing circuit connector 3-1 are connected to the patient end of the breathing circuit respectively, while the machine end of the breathing circuit connects to the anesthesia machine or ventilator. The endotracheal tube connector 2-3 of the endotracheal tube component 2 connects to the patient end of the breathing circuit connector 3-1, and after the inner tube body 4-1 is inserted through the second internal channel 3-1-4 of the machine end of the breathing circuit connector 3-1, the inner tube connector 4-3 of the inner tube component 4 and the machine end outer connector 3-1-10 of the breathing circuit connector 3-1 are respectively connected to the patient end of the breathing circuit, while the machine end of the breathing circuit connects to the anesthesia machine or ventilator; As the inner tube body 4-1 is gradually inserted, the apparatus dead space within the airway management system gradually decreases. When the patient end of the inner tube component 4 is positioned at the patient end of the larger diameter portion of the endotracheal tube 2-2, part of the apparatus dead space is eliminated.

The endotracheal tube 2-2 and endotracheal tube connector 2-3 or Laryngeal mask airway tube 1-2 and laryngeal mask connector 1-3 of this application have larger internal diameters than existing products, resulting in lower artificial airway resistance compared to existing products when patients have large spontaneous breathing tidal volumes. This application allows for the insertion of an inner tube component 4 through the endotracheal tube 2-2 and endotracheal tube connector 2-3 or through the Laryngeal mask airway tube 1-2 and laryngeal mask connector 1-3, which can reduce or even eliminate apparatus dead space. After inserting the inner tube component 4, the third ventilation cavity 4-2 of the inner tube component 4 and the second ventilation cavity 2-6 of the endotracheal tube component 2, or the third ventilation cavity 4-2 of the inner tube component 4 and the first ventilation cavity 1-6 of the laryngeal mask component 1, respectively form inhalation and exhalation cavities that can be interchanged. Which cavity serves as the inhalation cavity and which serves as the exhalation cavity can be determined according to clinical needs. The patient end of the gas sampling cavity is closer to the alveoli and is not affected by dead space gas, making sampling analysis more sensitive and accurate. The laryngeal mask connector 1-3 can connect to the patient end of the telescopic tube component 6. When the telescopic tube component 6 is fully extended to its completely open state, a stopper 6-4 is placed between the annular protrusions 6-5 of the telescopic tube component 6. The inner tube component 4 can be inserted through the laryngeal mask component 1 and the telescopic tube component 6, which can eliminate the apparatus dead space. After removing the stopper 6-4, as the telescopic tube component 6 gradually contracts, the patient end of the inner tube component 4 moves toward the patient's lungs, passing through the patient's glottis and entering the patient's trachea; When the telescopic tube component 6 is contracted to its original state, the patient end of the inner tube component 4 reaches the patient's trachea, eliminating all apparatus dead space and part of the anatomic dead space.

The embodiments described in the drawings of this application are exemplary, only used to explain this application, and should not be understood as limitations on this application. The invention is defined by the claims which follow:

## Claims

1. An airway management system, comprising a laryngeal mask component (1), an inner tube component (4) and a breathing circuit connector component (3);
wherein:
the laryngeal mask component (1) comprises a mask body (1-1) and a laryngeal mask airway tube (1-2) connected to one end of the mask body (1-1), and a first ventilation cavity (1-6) is arranged inside the laryngeal mask airway tube (1-2);
the laryngeal mask component (1) further comprises a laryngeal mask connector (1-3) connected to an end of the laryngeal mask airway tube (1-2) opposite to the mask body (1-1);
the inner tube component (4) comprises an inner tube body (4-1) and an inner tube connector (4-3) connected to the inner tube body (4-1), and a third ventilation cavity (4-2) is arranged inside the inner tube body (4-1);
the first ventilation cavity (1-6) is configured for inserting the inner tube component (4);
wherein the inner tube body (4-1) is configured to be inserted through the machine end second inner connector (3-1-9) and the first ventilation cavity (1-6);
as the inner tube body (4-1) is gradully inserted, a patient end of the inner tube component (4) is positioned within the mask body (1-1) and aligned with the patient end of the laryngeal mask airway tube (1-2), and as the inner tube body (4-1) is further inserted, the patient end of the inner tube component (4) passes through the glottis of a patient and to enter the trachea of the patient;
after the inner tube component (4) is inserted, one of the first ventilation cavity (1-6) and the third ventilation cavity (4-2) forms an inhalation cavity, and the other forms an exhalation cavity;
the breathing circuit connector component (3) comprises a breathing circuit connector (3-1), one end of the breathing circuit connector (3-1) being connected to the laryngeal mask connector (1-3), or being connected to the laryngeal mask connector (1-3) and the inner tube connector (4-3), and another end of the breathing circuit connector (3-1) being configured to be connected to an anesthesia machine or a ventilator through a breathing circuit;
the breathing circuit connector (3-1) comprises a patient end and a machine end, the patient end comprising a patient end inner connector (3-1-6) and a patient end outer connector (3-1-7), the patient end outer connector (3-1-7) being configured to connect to the laryngeal mask connector (1-3), and the patient end inner connector (3-1-6) being configured to connect to the inner tube connector (4-3);
the machine end comprises a machine end first inner connector (3-1-8), a machine end second inner connector (3-1-9) and a machine end outer connector (3-1-10), and three channels are provided in the machine end, the three channels being a machine end first inner channel (3-1-3), a machine end second inner channel (3-1-4) and a machine end outer channel (3-1-5), respectively;
the patient end comprises a patient end inner channel (3-1-1) and a patient end outer channel (3-1-2);
the machine end first inner channel (3-1-3) and the machine end second inner channel (3-1-4) are connected to the patient end inner channel (3-1-1), and the machine end outer channel (3-1-5) is connected to the patient end outer channel (3-1-2);
the patient end inner channel (3-1-1) and the patient end outer channel (3-1-2) are of a coaxial inner and outer dual-channel structure; and
the machine end first inner connector (3-1-8) and the machine end outer connector (3-1-10), or the machine end second inner connector (3-1-9) and the machine end outer connector (3-1-10), are configured to be connected to the anesthesia machine or the ventilator through the breathing circuit.

2. An airway management system, comprising a laryngeal mask component (1), an inner tube component (4), a breathing circuit connector component (3) and a telescopic tube component (6); wherein:
the laryngeal mask component (1) comprises a mask body (1-1) and a laryngeal mask airway tube (1-2) connected to one end of the mask body (1-1), and a first ventilation cavity (1-6) is arranged inside the laryngeal mask airway tube (1-2);
the laryngeal mask component (1) further comprises a laryngeal mask connector (1-3) connected to an end of the laryngeal mask airway tube (1-2) opposite to the mask body (1-1);
the inner tube component (4) comprises an inner tube body (4-1) and an inner tube connector (4-3) connected to the inner tube body (4-1), and a third ventilation cavity (4-2) is arranged inside the inner tube body (4-1);
the telescopic tube component (6) comprises a telescopic tube patient end connector (6-2), a telescopic tube machine end connector (6-3) and a telescopic tube body (6-1), one end of the telescopic tube body (6-1) is connected to the telescopic tube patient end connector (6-2), and the other end of the telescopic tube body (6-1) is connected to the telescopic tube machine end connector (6-3);
the telescopic tube patient end connector (6-2) is configured to connect to the laryngeal mask connector (1-3);
the inner tube component (4) is configured to be inserted via the laryngeal mask airway tube (1-2), the laryngeal mask connector (1-3) and the telescopic tube component (6);
when the telescopic tube component (6) is fully extended to its completely open state, a patient end of the inner tube component (4) is positioned within the mask body (1-1) and aligned with a patient end of the laryngeal mask airway tube (1-2);
as the telescopic tube component (6) gradually contracts from the completely open state, the patient end of the inner tube component (4) moves towards the lungs of a patient, passes through the glottis of the patient and enters the trachea of the patient;
after the inner tube component (4) is inserted, one of the first ventilation cavity (1-6) and the third ventilation cavity (4-2) forms an inhalation cavity, and the other forms an exhalation cavity;
the breathing circuit connector component (3) comprises a breathing circuit connector (3-1), one end of the breathing circuit connector (3-1) being connected to the telescopic tube machine end connector (6-3) and the inner tube connector (4-3), and another end of the breathing circuit connector (3-1) being configured to be connected to an anesthesia machine or a ventilator through a breathing circuit;
the breathing circuit connector (3-1) comprises a patient end and a machine end, the patient end comprising a patient end inner connector (3-1-6) and a patient end outer connector (3-1-7), the patient end outer connector (3-1-7) being configured to connect to the telescopic tube machine end connector (6-3), and the patient end inner connector (3-1-6) being configured to connect to the inner tube connector (4-3);
the machine end comprises a machine end first inner connector (3-1-8), a machine end second inner connector (3-1-9) and a machine end outer connector (3-1-10), and three channels are provided in the machine end, the three channels being a machine end first inner channel (3-1-3), a machine end second inner channel (3-1-4) and a machine end outer channel (3-1-5), respectively;
the patient end comprises a patient end inner channel (3-1-1) and a patient end outer channel (3-1-2);
the machine end first inner channel (3-1-3) and the machine end second inner channel (3-1-4) are connected to the patient end inner channel (3-1-1), and the machine end outer channel (3-1-5) is connected to the patient end outer channel (3-1-2);
the patient end inner channel (3-1-1) and the patient end outer channel (3-1-2) are of a coaxial inner and outer dual-channel structure; and
the machine end first inner connector (3-1-8) and the machine end outer connector (3-1-10), or the machine end second inner connector (3-1-9) and the machine end outer connector (3-1-10), are configured to be connected to the anesthesia machine or the ventilator through the breathing circuit.

3. The airway management system according to claim 1 or 2, wherein an openable breathing circuit connector top end cover (3-2) is provided at a top end of the machine end second inner connector (3-1-9), and a first Luer taper (3-3) is connected to a middle of the breathing circuit connector top end cover (3-2), and the breathing circuit connector top end cover (3-2) is configured to be connected to the machine end first inner connector (3-1-8).

4. The airway management system according to claim 2, wherein the telescopic tube component (6) further comprises annular protrusions (6-5) and a stopper (6-4);
the annular protrusions (6-5) are provided on outer side walls of the telescopic tube patient end connector (6-2) and the telescopic tube machine end connector (6-3), respectively;
the stopper (6-4) is configured to limit the telescopic tube component (6); and
when the telescopic tube component (6) is fully extended to its completely open state, the stopper (6-4) is located between the annular protrusions (6-5).

5. The airway management system according to claim 1 or 2, wherein a first gas sampling cavity (1-8) and a first throat aspiration cavity (1-7) are further provided in the laryngeal mask airway tube (1-2), wherein a patient end of the first gas sampling cavity (1-8) and a patient end of the first throat aspiration cavity (1-7) are close to a patient end of the laryngeal mask airway tube (1-2), and the first gas sampling cavity (1-8) and the first throat aspiration cavity (1-7) inside the laryngeal mask airway tube (1-2) are fixedly mated.

6. The airway management system according to claim 1 or 2, wherein the airway management system further comprises a connecting tube (5), and the connecting tube (5) is of an unequal diameter structure, wherein the connecting tube (5) comprises a connecting tube body (5-1);
when clinical anesthesia induction is performed, one end of the connecting tube body (5-1) is configured to be connected to the patient end of the breathing circuit connector (3-1), and the other end of the connecting tube body (5-1) is configured to be connected to an anesthetic mask; and
the connecting tube (5) further comprises a second Luer taper (5-2) configured to be connected to a gas sampling line of a monitor.

7. The airway management system according to claim 1 or 2, wherein an outer wall of the inner tube body (4-1) is provided with a plurality of protrusions (4-4) configured for positioning between the inner tube body (4-1) and the first ventilation cavity (1-6).

8. The airway management system according to claim 1 or 2, wherein a drainage cavity (1-10) and/or a video component cavity (1-9) is further provided in the laryngeal mask airway tube (1-2).

## Patentansprüche

1. Atemwegsmanagementsystem, umfassend eine Larynxmaskenkomponente (1), eine Innenschlauchkomponente (4) und eine Atemkreislaufanschlusskomponente (3),
wobei:
die Larynxmaskenkomponente (1) einen Maskenkörper (1-1) und einen mit einem Ende des Maskenkörpers (1-1) verbundenen Larynxmasken-Atemwegsschlauch (1-2) umfasst und im Inneren des Larynxmasken-Atemwegsschlauchs (1-2) ein erster Beatmungshohlraum (1-6) angeordnet ist;
die Larynxmaskenkomponente (1) ferner einen Larynxmaskenanschluss (1-3) umfasst, der mit einem dem Maskenkörper (1-1) gegenüberliegenden Ende des Larynxmasken-Atemwegsschlauchs (1-2) verbunden ist;
die Innenschlauchkomponente (4) einen Innenschlauchkörper (4-1) und einen mit dem Innenschlauchkörper (4-1) verbundenen Innenschlauchanschluss (4-3) umfasst und im Inneren des Innenschlauchkörpers (4-1) ein dritter Beatmungshohlraum (4-2) angeordnet ist;
der erste Beatmungshohlraum (1-6) zum Einführen der Innenschlauchkomponente (4) ausgebildet ist;
der Innenschlauchkörper (4-1) dazu ausgebildet ist, durch den maschinenseitigen zweiten Innenanschluss (3-1-9) und den ersten Beatmungshohlraum (1-6) eingefü hrt zu werden;
beim allmählichen Einführen des Innenschlauchkörpers (4-1) ein patientenseitiges Ende der Innenschlauchkomponente (4) innerhalb des Maskenkörpers (1-1) positioniert und auf das patientenseitige Ende des Larynxmasken-Atemwegsschlauchs (1-2) ausgerichtet wird und beim weiteren Einführen des Innenschlauchkörpers (4-1) das patientenseitige Ende der Innenschlauchkomponente (4) durch die Stimmritze eines Patienten hindurchtritt und in die Luftröhre des Patienten eintritt;
nach dem Einführen der Innenschlauchkomponente (4) einer von dem ersten Beatmungshohlraum (1-6) und dem dritten Beatmungshohlraum (4-2) einen Einatmungshohlraum bildet und der andere einen Ausatmungshohlraum bildet;
die Atemkreislaufanschlusskomponente (3) einen Atemkreislaufanschluss (3-1) umfasst, wobei ein Ende des Atemkreislaufanschlusses (3-1) mit dem Larynxmaskenanschluss (1-3) oder mit dem Larynxmaskenanschluss (1-3) und dem Innenschlauchanschluss (4-3) verbunden ist und ein anderes Ende des Atemkreislaufanschlusses (3-1) dazu ausgebildet ist, über einen Atemkreislauf mit einem Anästhesiegerät oder einem Beatmungsgerät verbunden zu werden;
der Atemkreislaufanschluss (3-1) ein patientenseitiges Ende und ein maschinenseitiges Ende umfasst, wobei das patientenseitige Ende einen patientenseitigen Innenanschluss (3-1-6) und einen patientenseitigen Außenanschluss (3-1-7) umfasst, wobei der patientenseitige Außenanschluss (3-1-7) zur Verbindung mit dem Larynxmaskenanschluss (1-3) ausgebildet ist und der patientenseitige Innenanschluss (3-1-6) zur Verbindung mit dem Innenschlauchanschluss (4-3) ausgebildet ist;
das maschinenseitige Ende einen maschinenseitigen ersten Innenanschluss (3-1-8), einen maschinenseitigen zweiten Innenanschluss (3-1-9) und einen maschinenseitigen Außenanschluss (3-1-10) umfasst, wobei in dem maschinenseitigen Ende drei Kanäle vorgesehen sind, nämlich ein maschinenseitiger erster Innenkanal (3-1-13), ein maschinenseitiger zweiter Innenkanal (3-1-4) und ein maschinenseitiger Außenkanal (3-1-5);
das patientenseitige Ende einen patientenseitigen Innenkanal (3-1-1) und einen patientenseitigen Außenkanal (3-1-2) umfasst;
der maschinenseitige erste Innenkanal (3-1-3) und der maschinenseitige zweite Innenkanal (3-1-4) mit dem patientenseitigen Innenkanal (3-1-1) verbunden sind und der maschinenseitige Außenkanal (3-1-5) mit dem patientenseitigen Außenkanal (3-1-2) verbunden ist;
der patientenseitige Innenkanal (3-1-1) und der patientenseitige Außenkanal (3-1-2) eine koaxiale Zweikanalstruktur mit einem inneren und einem äußeren Kanal aufweisen; und
der maschinenseitige erste Innenanschluss (3-1-8) und der maschinenseitige Außenanschluss (3-1-10) oder der maschinenseitige zweite Innenanschluss (3-1-9) und der maschinenseitige Außenanschluss (3-1-10) dazu ausgebildet sind, über den Atemkreislauf mit dem Anästhesiegerät oder dem Beatmungsgerät verbunden zu werden.

2. Atemwegsmanagementsystem, umfassend eine Larynxmaskenkomponente (1), eine Innenschlauchkomponente (4), eine Atemkreislaufanschlusskomponente (3) und eine Teleskopschlauchkomponente (6),
wobei:
die Larynxmaskenkomponente (1) einen Maskenkörper (1-1) und einen mit einem Ende des Maskenkörpers (1-1) verbundenen Larynxmasken-Atemwegsschlauch (1-2) umfasst und im Inneren des Larynxmasken-Atemwegsschlauchs (1-2) ein erster Beatmungshohlraum (1-6) angeordnet ist;
die Larynxmaskenkomponente (1) ferner einen Larynxmaskenanschluss (1-3) umfasst, der mit einem dem Maskenkörper (1-1) gegenüberliegenden Ende des Larynxmasken-Atemwegsschlauchs (1-2) verbunden ist;
die Innenschlauchkomponente (4) einen Innenschlauchkörper (4-1) und einen mit dem Innenschlauchkörper (4-1) verbundenen Innenschlauchanschluss (4-3) umfasst und im Inneren des Innenschlauchkörpers (4-1) ein dritter Beatmungshohlraum (4-2) angeordnet ist;
die Teleskopschlauchkomponente (6) einen patientenseitigen Teleskopschlauchanschluss (6-2), einen maschinenseitigen Teleskopschlauchanschluss (6-3) und einen Teleskopschlauchkörper (6-1) umfasst, wobei ein Ende des Teleskopschlauchkörpers (6-1) mit dem patientenseitigen Teleskopschlauchanschluss (6-2) verbunden ist und das andere Ende des Teleskopschlauchkörpers (6-1) mit dem maschinenseitigen Teleskopschlauchanschluss (6-3) verbunden ist;
der patientenseitige Teleskopschlauchanschluss (6-2) zur Verbindung mit dem Larynxmaskenanschluss (1-3) ausgebildet ist;
die Innenschlauchkomponente (4) dazu ausgebildet ist, über den Larynxmasken-Atemwegsschlauch (1-2), den Larynxmaskenanschluss (1-3) und die Teleskopschlauchkomponente (6) eingeführt zu werden;
wenn die Teleskopschlauchkomponente (6) vollständig in ihren vollständig geöffneten Zustand ausgezogen ist, ein patientenseitiges Ende der Innenschlauchkomponente (4) innerhalb des Maskenkörpers (1-1) positioniert und auf ein patientenseitiges Ende des Larynxmasken-Atemwegsschlauchs (1-2) ausgerichtet ist;
wenn sich die Teleskopschlauchkomponente (6) ausgehend von dem vollständig geöffneten Zustand allmählich zusammenzieht, sich das patientenseitige Ende der Innenschlauchkomponente (4) in Richtung der Lunge eines Patienten bewegt, durch die Stimmritze des Patienten hindurchtritt und in die Luftröhre des Patienten eintritt;
nach dem Einführen der Innenschlauchkomponente (4) einer von dem ersten Beatmungshohlraum (1-6) und dem dritten Beatmungshohlraum (4-2) einen Einatmungshohlraum bildet und der andere einen Ausatmungshohlraum bildet;
die Atemkreislaufanschlusskomponente (3) einen Atemkreislaufanschluss (3-1) umfasst, wobei ein Ende des Atemkreislaufanschlusses (3-1) mit dem maschinenseitigen Teleskopschlauchanschluss (6-3) und dem Innenschlauchanschluss (4-3) verbunden ist und ein anderes Ende des Atemkreislaufanschlusses (3-1) dazu ausgebildet ist, über einen Atemkreislauf mit einem Anästhesiegerät oder einem Beatmungsgerät verbunden zu werden;
der Atemkreislaufanschluss (3-1) ein patientenseitiges Ende und ein maschinenseitiges Ende umfasst, wobei das patientenseitige Ende einen patientenseitigen Innenanschluss (3-1-6) und einen patientenseitigen Außenanschluss (3-1-7) umfasst, wobei der patientenseitige Außenanschluss (3-1-7) zur Verbindung mit dem maschinenseitigen Teleskopschlauchanschluss (6-3) ausgebildet ist und der patientenseitige Innenanschluss (3-1-6) zur Verbindung mit dem Innenschlauchanschluss (4-3) ausgebildet ist;
das maschinenseitige Ende einen maschinenseitigen ersten Innenanschluss (3-1-8), einen maschinenseitigen zweiten Innenanschluss (3-1-9) und einen maschinenseitigen Außenanschluss (3-1-10) umfasst, wobei in dem maschinenseitigen Ende drei Kanäle vorgesehen sind, nämlich ein maschinenseitiger erster Innenkanal (3-1-3), ein maschinenseitiger zweiter Innenkanal (3-1-4) und ein maschinenseitiger Außenkanal (3-1-5);
das patientenseitige Ende einen patientenseitigen Innenkanal (3-1-1) und einen patientenseitigen Außenkanal (3-1-2) umfasst;
der maschinenseitige erste Innenkanal (3-1-3) und der maschinenseitige zweite Innenkanal (3-1-4) mit dem patientenseitigen Innenkanal (3-1-1) verbunden sind und der maschinenseitige Außenkanal (3-1-5) mit dem patientenseitigen Außenkanal (3-1-2) verbunden ist;
der patientenseitige Innenkanal (3-1-1) und der patientenseitige Außenkanal (3-1-2) eine koaxiale Zweikanalstruktur mit einem inneren und einem äußeren Kanal aufweisen; und
der maschinenseitige erste Innenanschluss (3-1-8) und der maschinenseitige Außenanschluss (3-1-10) oder der maschinenseitige zweite Innenanschluss (3-1-9) und der maschinenseitige Außenanschluss (3-1-10) dazu ausgebildet sind, über den Atemkreislauf mit dem Anästhesiegerät oder dem Beatmungsgerät verbunden zu werden.

3. Atemwegsmanagementsystem nach Anspruch 1 oder 2, wobei an einem oberen Ende des maschinenseitigen zweiten Innenanschlusses (3-1-9) eine öffenbare obere Endabdeckung des Atemkreislaufanschlusses (3-2) vorgesehen ist, wobei ein erster Luer-Konus (3-3) mit einem mittleren Bereich der oberen Endabdeckung des Atemkreislaufanschlusses (3-2) verbunden ist und wobei die obere Endabdeckung des Atemkreislaufanschlusses (3-2) dazu ausgebildet ist, mit dem maschinenseitigen ersten Innenanschluss (3-1-8) verbunden zu werden.

4. Atemwegsmanagementsystem nach Anspruch 2, wobei die Teleskopschlauchkomponente (6) ferner ringförmige Vorsprünge (6-5) und ein Anschlagelement (6-4) umfasst;
wobei die ringförmigen Vorsprünge (6-5) jeweils an Außenwänden des patientenseitigen Teleskopschlauchanschlusses (6-2) und des maschinenseitigen Teleskopschlauchanschlusses (6-3) vorgesehen sind;
wobei das Anschlagelement (6-4) zur Begrenzung der Teleskopschlauchkomponente (6) ausgebildet ist; und
wobei sich das Anschlagelement (6-4) zwischen den ringförmigen Vorsprüngen (6-5) befindet, wenn die Teleskopschlauchkomponente (6) vollständig in ihren vollständig geöffneten Zustand ausgezogen ist.

5. Atemwegsmanagementsystem nach Anspruch 1 oder 2, wobei in dem Larynxmasken-Atemwegsschlauch (1-2) ferner ein erster Gasprobenentnahmehohlraum (1-8) und ein erster Rachenabsaughohlraum (1-7) vorgesehen sind, wobei sich ein patientenseitiges Ende des ersten Gasprobenentnahmehohlraums (1-8) und ein patientenseitiges Ende des ersten Rachenabsaughohlraums (1-7) nahe einem patientenseitigen Ende des Larynxmasken-Atemwegsschlauchs (1-2) befinden und der erste Gasprobenentnahmehohlraum (1-8) und der erste Rachenabsaughohlraum (1-7) innerhalb des Larynxmasken-Atemwegsschlauchs (1-2) fest miteinander zusammengefügt sind.

6. Atemwegsmanagementsystem nach Anspruch 1 oder 2, wobei das Atemwegsmanagementsystem ferner ein Verbindungsrohr (5) umfasst und das Verbindungsrohr (5) eine Struktur mit unterschiedlichen Durchmessern aufweist, wobei das Verbindungsrohr (5) einen Verbindungsrohrkörper (5-1) umfasst;
wobei bei Durchführung einer klinischen Narkoseeinleitung ein Ende des Verbindungsrohrkörpers (5-1) dazu ausgebildet ist, mit dem patientenseitigen Ende des Atemkreislaufanschlusses (3-1) verbunden zu werden, und das andere Ende des Verbindungsrohrkörpers (5-1) dazu ausgebildet ist, mit einer Anästhesiemaske verbunden zu werden; und
wobei das Verbindungsrohr (5) ferner einen zweiten Luer-Konus (5-2) umfasst, der zur Verbindung mit einer Gasprobenentnahmeleitung eines Überwachungsgeräts ausgebildet ist.

7. Atemwegsmanagementsystem nach Anspruch 1 oder 2, wobei eine Außenwand des Innenschlauchkörpers (4-1) mit einer Mehrzahl von Vorsprüngen (4-4) versehen ist, die zur Positionierung zwischen dem Innenschlauchkörper (4-1) und dem ersten Beatmungshohlraum (1-6) ausgebildet sind.

8. Atemwegsmanagementsystem nach Anspruch 1 oder 2, wobei in dem Larynxmasken-Atemwegsschlauch (1-2) ferner ein Drainagehohlraum (1-10) und/oder ein Videokomponentenhohlraum (1-9) vorgesehen ist.

## Revendications

1. Système de prise en charge de voies respiratoires, comprenant un composant de masque laryngé (1), un composant de tube interne (4) et un composant de raccord de circuit respiratoire (3) ;
dans lequel :
le composant de masque laryngé (1) comprend un corps de masque (1-1) et un tube respiratoire de masque laryngé (1-2) raccordé à une extrémité du corps de masque (1-1), et une première cavité de ventilation (1-6) est disposée à l'intérieur du tube respiratoire de masque laryngé (1-2) ;
le composant de masque laryngé (1) comprend en outre un raccord de masque laryngé (1-3) raccordé à une extrémité du tube respiratoire de masque laryngé (1-2) opposée au corps de masque (1-1) ;
le composant de tube interne (4) comprend un corps de tube interne (4-1) et un raccord de tube interne (4-3) raccordé au corps de tube interne (4-1), et une troisième cavité de ventilation (4-2) est disposée à l'intérieur du corps de tube interne (4-1) ;
la première cavité de ventilation (1-6) est conçue pour insérer le composant de tube interne (4) ;
dans lequel le corps de tube interne (4-1) est conçu pour être inséré à travers le second raccord interne d'extrémité appareil (3-1-9) et la première cavité de ventilation (1-6) ;
à mesure que le corps de tube interne (4-1) est inséré progressivement, une extrémité patient du composant de tube interne (4) est positionnée à l'intérieur du corps de masque (1-1) et alignée avec l'extrémité patient du tube respiratoire de masque laryngé (1-2), et à mesure que le corps de tube interne (4-1) est inséré davantage, l'extrémité patient du composant de tube interne (4) passe à travers la glotte d'un patient et pénètre dans la trachée du patient ;
une fois le composant de tube interne (4) inséré, l'une de la première cavité de ventilation (1-6) et de la troisième cavité de ventilation (4-2) forme une cavité d'inspiration, et l'autre forme une cavité d'expiration ;
le composant de raccord de circuit respiratoire (3) comprend un raccord de circuit respiratoire (3-1), une extrémité du raccord de circuit respiratoire (3-1) étant raccordée au raccord de masque laryngé (1-3), ou étant raccordée au raccord de masque laryngé (1-3) et au raccord de tube interne (4-3), et l'autre extrémité du raccord de circuit respiratoire (3-1) étant conçue pour être raccordée à un appareil d'anesthésie ou à un ventilateur par l'intermédiaire d'un circuit respiratoire ;
le raccord de circuit respiratoire (3-1) comprend une extrémité patient et une extrémité appareil, l'extrémité patient comprenant un raccord interne d'extrémité patient (3-1-6) et un raccord externe d'extrémité patient (3-1-7), le raccord externe d'extrémité patient (3-1-7) étant conçu pour être raccordé au raccord de masque laryngé (1-3), et le raccord interne d'extrémité patient (3-1-6) étant conçu pour être raccordé au raccord de tube interne (4-3) ;
l'extrémité appareil comprend un premier raccord interne d'extrémité appareil (3-1-8), un second raccord interne d'extrémité appareil (3-1-9) et un raccord externe d'extrémité appareil (3-1-10), et trois canaux sont prévus dans l'extrémité appareil, les trois canaux étant respectivement un premier canal interne d'extrémité appareil (3-1-3), un second canal interne d'extrémité appareil (3-1-4) et un canal externe d'extrémité appareil (3-1-5) ;
l'extrémité patient comprend un canal interne d'extrémité patient (3-1-1) et un canal externe d'extrémité patient (3-1-2) ;
le premier canal interne d'extrémité appareil (3-1-3) et le second canal interne d'extrémité appareil (3-1-4) sont raccordés au canal interne d'extrémité patient (3-1-1), et le canal externe d'extrémité appareil (3-1-5) est raccordé au canal externe d'extrémité patient (3-1-2) ;
le canal interne d'extrémité patient (3-1-1) et le canal externe d'extrémité patient (3-1-2) présentent une structure à double canal interne et externe coaxial ; et
le premier raccord interne d'extrémité appareil (3-1-8) et le raccord externe d'extrémité appareil (3-1-10), ou le second raccord interne d'extrémité appareil (3-1-9) et le raccord externe d'extrémité appareil (3-1-10), sont conçus pour être raccordés à l'appareil d'anesthésie ou au ventilateur par l'intermédiaire du circuit respiratoire.

2. Système de prise en charge de voies respiratoires, comprenant un composant de masque laryngé (1), un composant de tube interne (4), un composant de raccord de circuit respiratoire (3) et un composant de tube télescopique (6) ; dans lequel :
le composant de masque laryngé (1) comprend un corps de masque (1-1) et un tube respiratoire de masque laryngé (1-2) raccordé à une extrémité du corps de masque (1-1), et une première cavité de ventilation (1-6) est disposée à l'intérieur du tube respiratoire de masque laryngé (1-2) ;
le composant de masque laryngé (1) comprend en outre un raccord de masque laryngé (1-3) raccordé à une extrémité du tube respiratoire de masque laryngé (1-2) opposée au corps de masque (1-1) ;
le composant de tube interne (4) comprend un corps de tube interne (4-1) et un raccord de tube interne (4-3) raccordé au corps de tube interne (4-1), et une troisième cavité de ventilation (4-2) est disposée à l'intérieur du corps de tube interne (4-1) ;
le composant de tube télescopique (6) comprend un raccord d'extrémité patient de tube télescopique (6-2), un raccord d'extrémité appareil de tube télescopique (6-3) et un corps de tube télescopique (6-1), une extrémité du corps de tube télescopique (6-1) est raccordée au raccord d'extrémité patient de tube télescopique (6-2), et l'autre extrémité du corps de tube télescopique (6-1) est raccordée au raccord d'extrémité appareil de tube télescopique (6-3) ;
le raccord d'extrémité patient de tube télescopique (6-2) est conçu pour être raccordé au raccord de masque laryngé (1-3) ;
le composant de tube interne (4) est conçu pour être inséré par l'intermédiaire du tube respiratoire de masque laryngé (1-2), du raccord de masque laryngé (1-3) et du composant de tube télescopique (6) ;
lorsque le composant de tube télescopique (6) s'étend entièrement jusqu'à son état complètement ouvert, une extrémité patient du composant de tube interne (4) est positionnée à l'intérieur du corps de masque (1-1) et alignée avec une extrémité patient du tube respiratoire de masque laryngé (1-2) ;
à mesure que le composant de tube télescopique (6) se rétracte progressivement à partir de son état complètement ouvert, l'extrémité patient du composant de tube interne (4) se déplace vers les poumons d'un patient, passe à travers la glotte du patient et pénètre dans la trachée de du patient ;
une fois le composant de tube interne (4) inséré, l'une de la première cavité de ventilation (1-6) et de la troisième cavité de ventilation (4-2) forme une cavité d'inspiration, et l'autre forme une cavité d'expiration ;
le composant de raccord de circuit respiratoire (3) comprend un raccord de circuit respiratoire (3-1), une extrémité du raccord de circuit respiratoire (3-1) étant raccordée au raccord d'extrémité appareil de tube télescopique (6-3) et au raccord de tube interne (4-3), et l'autre extrémité du raccord de circuit respiratoire (3-1) étant conçue pour être raccordée à un appareil d'anesthésie ou à un ventilateur par l'intermédiaire d'un circuit respiratoire ;
le raccord de circuit respiratoire (3-1) comprend une extrémité patient et une extrémité appareil, l'extrémité patient comprenant un raccord interne d'extrémité patient (3-1-6) et un raccord externe d'extrémité patient (3-1-7), le raccord externe d'extrémité patient (3-1-7) étant conçu pour être raccordé au raccord d'extrémité appareil de tube télescopique (6-3), et le raccord interne d'extrémité patient (3-1-6) étant conçu pour être raccordé au raccord de tube interne (4-3) ;
l'extrémité appareil comprend un premier raccord interne d'extrémité appareil (3-1-8), un second raccord interne d'extrémité appareil (3-1-9) et un raccord externe d'extrémité appareil (3-1-10), et trois canaux sont prévus dans l'extrémité appareil, les trois canaux étant respectivement un premier canal interne d'extrémité appareil (3-1-3), un second canal interne d'extrémité appareil (3-1-4) et un canal externe d'extrémité appareil (3-1-5) ;
l'extrémité patient comprend un canal interne d'extrémité patient (3-1-1) et un canal externe d'extrémité patient (3-1-2) ;
le premier canal interne d'extrémité appareil (3-1-3) et le second canal interne d'extrémité appareil (3-1-4) sont raccordés au canal interne d'extrémité patient (3-1-1), et le canal externe d'extrémité appareil (3-1-5) est raccordé au canal externe d'extrémité patient (3-1-2) ;
le canal interne d'extrémité patient (3-1-1) et le canal externe d'extrémité patient (3-1-2) présentent une structure à double canal interne et externe coaxial ; et
le premier raccord interne d'extrémité appareil (3-1-8) et le raccord externe d'extrémité appareil (3-1-10), ou le second raccord interne d'extrémité appareil (3-1-9) et le raccord externe d'extrémité appareil (3-1-10), sont conçus pour être raccordés à l'appareil d'anesthésie ou au ventilateur par l'intermédiaire du circuit respiratoire.

3. Système de prise en charge de voies respiratoires selon la revendication 1 ou 2, dans lequel un capot d'extrémité supérieure (3-2) pouvant être ouvert du raccord de circuit respiratoire est prévu au niveau d'une extrémité supérieure du second raccord interne d'extrémité appareil (3-1-9), et un premier cône Luer (3-3) est raccordé à une partie centrale du capot d'extrémité supérieure (3-2) du raccord de circuit respiratoire, et le capot d'extrémité supérieure (3-2) du raccord de circuit respiratoire est conçu pour être raccordé au premier raccord interne d'extrémité appareil (3-1-8).

4. Système de prise en charge de voies respiratoires selon la revendication 2, dans lequel le composant de tube télescopique (6) comprend en outre des saillies annulaires (6-5) et une butée (6-4) ;
les saillies annulaires (6-5) sont prévues respectivement sur des parois latérales externes du raccord d'extrémité patient de tube télescopique (6-2) et du raccord d'extrémité appareil de tube télescopique (6-3) ;
la butée (6-4) est conçue pour limiter la course du composant de tube télescopique (6) ; et
lorsque le composant de tube télescopique (6) s'étend entièrement jusqu'à son état complètement ouvert, la butée (6-4) est située entre les saillies annulaires (6-5).

5. Système de prise en charge de voies respiratoires selon la revendication 1 ou 2, dans lequel une première cavité de prélèvement de gaz (1-8) et une première cavité d'aspiration de gorge (1-7) sont en outre prévues dans le tube respiratoire de masque laryngé (1-2), dans lequel une extrémité patient de la première cavité de prélèvement de gaz (1-8) et une extrémité patient de la première cavité d'aspiration de gorge (1-7) sont proches d'une extrémité patient du tube respiratoire de masque laryngé (1-2), et la première cavité de prélèvement de gaz (1-8) et la première cavité d'aspiration de gorge (1-7) à l'intérieur du tube respiratoire de masque laryngé (1-2) sont assemblées de manière fixe.

6. Système de prise en charge de voies respiratoires selon la revendication 1 ou 2, dans lequel le système de prise en charge de voies respiratoires comprend en outre un tube de raccordement (5), et le tube de raccordement (5) présente une structure à diamètres inégaux, dans lequel le tube de raccordement (5) comprend un corps de tube de raccordement (5-1) ;
lorsqu'une induction anesthésique clinique est effectuée, une extrémité du corps de tube de raccordement (5-1) est conçue pour être raccordée à l'extrémité patient du raccord de circuit respiratoire (3-1), et l'autre extrémité du corps de tube de raccordement (5-1) est conçue pour être raccordée à un masque d'anesthésie ; et
le tube de raccordement (5) comprend en outre un second cône Luer (5-2) conçu pour être raccordé à une ligne de prélèvement de gaz d'un moniteur.

7. Système de prise en charge de voies respiratoires selon la revendication 1 ou 2, dans lequel une paroi externe du corps de tube interne (4-1) est pourvue d'une pluralité de saillies (4-4) conçues pour se positionner entre le corps de tube interne (4-1) et la première cavité de ventilation (1-6).

8. Système de prise en charge de voies respiratoires selon la revendication 1 ou 2, dans lequel une cavité de drainage (1-10) et/ou une cavité de composant vidéo (1-9) sont en outre prévues dans le tube respiratoire de masque laryngé (1-2).
